# EUROPÄISCHE PATENTSCHRIFT

(11) **EP 3 253 326 B1**
(45) Veröffentlichungstag und Bekanntmachung des Hinweises auf die Patenterteilung: **29.12.2021**
(21) Anmeldenummer: 16702723.4
(22) Anmeldetag: 03.02.2016
(51) Int. Cl.: A61C 8/00, A61L 27/54, A61C 8/02, A61L 27/32

(54) **BESCHICHTUNG DENTALER, PROTHETISCHER OBERFLÄCHEN MIT EINER DEUTLICHEN SCHICHT EINES SYNTHETISCHEN HYDROXYAPATITS**
COATING OF DENTAL PROSTHETIC SURFACES COMPRISING A DISTINCT LAYER OF A SYNTHETIC HYDROXYAPATITE
REVÊTEMENT DE SURFACES PROTHÉTIQUES DENTAIRES, PRÉSENTANT UNE COUCHE SPÉCIFIQUE D'UNE HYDROXYAPATITE DE SYNTHÈSE

(30) Priorität: 04.02.2015 DE 102015101626
(43) Veröffentlichungstag der Anmeldung: 13.12.2017
(73) Patentinhaber: Kulzer GmbH, 63450 Hanau (DE)
(72) Erfinder: BALKENHOL, Markus, 63543 Neuberg (DE); HOFFMANN, Marcus, Dr., 61250 Usingen (DE); LEYER, Andrea, 63619 Bad Orb (DE); BUSCH, Susanne, 61273 Wehrheim (DE)
(74) Vertreter: Bendele, Tanja
(86) Internationale Anmeldenummer: PCT/EP2016/052286
(87) Internationale Veröffentlichungsnummer: WO 2016/124643

(56) Entgegenhaltungen:
- WO-A1-2013/011827
- US-A1- 2008 020 349
- US-B1- 6 214 049

## Beschreibung

Gegenstand der Erfindung sind prothetische Formteile, die zumindest bereichsweise an ihrer Oberfläche mindestens eine Schicht biomimetisches Apatit ausgewählt aus Fluorapatit, Hydroxylapatit oder deren Gemischen aufweisen, wobei die Oberflächen der Formteile mindestens in diesem Bereich mikromechanische Verankerungsstellen zur Verbesserung der mechanischen Anbindung von Apatit an die Oberfläche aufweisen. Ebenfalls Gegenstand der Erfindung sind Formteile zur Verwendung zur dentalen, prothetischen Versorgung bei Zahnverlust, insbesondere zur zellulären Anheftung von Zellen an prothetische Formteile, bevorzugt eine Anheftung von Zellen über Hemidesmosomen. Ferner ist Gegenstand der Erfindung das Verfahren zur Herstellung der prothetischen Formteile.

Ein Problem von festsitzendem, permanentem Zahnersatz, wie implantgetragenen prothetischen Versorgungen, ist häufig ein verbleibender Spalt zwischen dem Implantatpfeiler und dem umgebenden Zahnfleisch. Ausgehend von diesem Spalt werden immer wieder Infektionen des Knochens und des umgebenden Weichgewebes ausgelöst. Bekannt ist diese Problematik unter dem Fachbegriff Periimplantitis oder periimplantäre Infektion. Nach einer Implantation wird ein direkter funktioneller und struktureller Verbund zwischen dem Implantat und dem umgebenden Knochengewebe (Osteoblasten/ Knochenzellen) angestrebt, um das Risiko einer Infektion zu minimieren. Ein derart eingewachsenes Implantat ist osseointegriert. Daher soll die Adhäsion und Aktivität von Osteoblasten auf Implantatoberflächen gesteigert werden, um eine beschleunigte Implantatintegration zu erreichen. Eine Osseointegration eines Implantates kann mittels Ultraschall und Röntgen überprüft werden. Zudem ist ein osseointegriertes Implantat unbeweglich.

EP0125203 offenbart eine gesinterte, poröse Hydroxylapatithülse, die auf einen Implantatpfosten aufsetzbar ist. Ein Hülsenrohling wird aus gepresstem amorphen Material hergestellt, vermutlich einem Calciumphosphat (Ca₃(PO₄)₂) und sodann gesintert, um das amorphe Material in kristallines umzuwandeln. Es wird herausgestellt, dass ein Hineinwachsen des Knochengewebes und der Deckhaut in die poröse, kristalline Hydroxyapatitschicht erfolgt. Die gemäss dem Verfahren der EP0125203 hergestellten kristallinen Apatite weisen eine erheblich grössere Kristallgrösse als biomimetisch abgeschiedenes Apatit auf.

EP0657178A2 offenbart ein Verfahren zur Herstellung von Implantatkeramikmaterial mit Hydroxylapatit. Auch hier wird ein Verfahren offenbart, in dem das zu verwendende Material gesintert wird. Ausgangsmaterial ist das spongiöse Material im Bereich der Gelenke von Rindern. Das anorganische Material der Gelenke wird bei hohen Temperaturen von 650 bis 1250 °C gesintert. Die sehr hohen Temperaturen sind notwendig, um das Kristallwachstum zu beschleunigen.

WO2011/022642A1 betrifft nanoscalige Hydroxylapatitbeschichtungen (HAp) auf orthopädischen Implantaten in einer Mischung mit ZnO, die mittels ESC-Abscheidung aufgebracht werden. Das Dokument verweist auf eine Vielzahl von Versuchen HAp auf metallischen Oberflächen mit akzeptablen Eigenschaften einer dauerhaften Beschichtung herzustellen. Ein wesentlicher Nachteil von amorphem Calciumphosphat ist dessen Löslichkeit in Körperflüssigkeiten, so dass das HAp im Laufe der Zeit abgebaut wird. Das Verfahren der WO2011/022642 offenbart ein Verfahren umfassend die Mahlschritte mit einer Kugelmühle und "supersonic jet miling" einer Strahlenmühle. Die Abscheidung der nanopartikulären Teilchen erfolgt über ein Aufsprühen in einem elektrostatischen Feld (Electrospraying). Nachfolgend wird die Beschichtung gesintert.

WO2011/049915A2 offenbart eine elektrochemische Abscheidung von Hydroxylapatit aus einer wässrigen Lösung auf Implantaten. Das Verfahren ist auf elektrisch leitende Materialoberfläche beschränkt.

US 2008/020349 A1 offenbart ein Knochenimplantat mit einer osteogenen Schicht aus Calciumsulfat, welche zusätzlich einen Anteil an biomimetischen, natürlichem oder synthetischen Hydroxylapatit umfassen kann.

Aufgabe der vorliegenden Erfindung war die Bereitstellung von prothetischen Formteilen, die die Nachteile des Standes der Technik nicht aufweisen. Zudem bestand die Aufgabe prothetische Formteile bereitzustellen, die eine zelluläre Anheftung von Zellen des Zahnfleisches bzw. der Schleimhautzellen des Zahnfleisches über Hemidesmosomen oder andere biologische Mechanismen an die mit ihm in Kontakt tretenden Bereiche der Prothese erlauben. Daher bestand die Aufgabe darin prothetische Formteile bereitzustellen, die eine biologische Verbindung bzw. eine biologische Anheftung mit den sie umgebenden Zellen ermöglichen. Eine weitere Aufgabe bestand darin kristallinen, biomimetischen Apatit auf prothetischen Formteilen bereitzustellen, insbesondere auch auf nicht elektrisch leitenden Oberflächen, das eine biologische Verbindung mit den umgebenen Zellen eingehen kann, insbesondere mit Zahnfleisch oder Epithelzelle, Fibroblasten und/oder Osteoblasten.

Gelöst werden die Aufgaben der Erfindung durch ein prothetisches Formteil nach Anspruch 1 sowie ein Verfahren zur Herstellung des prothetischen Formteils nach Anspruch 9 und die Verwendung eines solchen prothetischen Formteils. Bevorzugte Ausführungsformen der Erfindung sind in den Unteransprüchen sowie in der Beschreibung detailliert offenbart. Prothetische Formteile gemäss der Erfindung umfassen auch chirurgische Formteile. Ganz besonders bevorzugte prothetische Formteile sind Implantate, Implantat-Aufbauten sowie alle Komponenten die mit dem Implantat verbunden werden können.

Gegenstand der Erfindung ist ein prothetisches Formteil, wobei das Formteil zumindest bereichsweise an seiner Oberfläche mindestens eine Schicht biomimetischen Apatit ausgewählt aus Fluorapatit, Hydroxylapatit oder deren Gemischen aufweist, wobei die Oberfläche mindestens in diesem Bereich mikromechanische Verankerungsstellen aufweist. Vorzugsweise weist die Oberfläche in diesem Bereich zudem chemische Verankerungsstellen auf, diese Oberfläche gilt dann als aktiviert. Als chemische Verankerungsstellen gelten Bereiche auf der Oberfläche, die die biomimetische Kristallisation von Apatit begünstigen, wie beispielsweise Hydroxy-Gruppen aufweisende Bereiche der Oberfläche.

Gegenstand der Erfindung ist ebenfalls ein Formteil bei dem der biomimetische Apatit mit den mikromechanischen Verankerungsstellen, insbesondere in Form einer porösen Oberfläche, mechanisch verwachsen ist. Gleichfalls kann es bevorzugt, sein wenn die Oberlfächen der prothetischen Formteile, insbesondere im Bereich der mikromechanischen Verankerungstellen mit TiN und/oder ZrN oder einem andere physiologisch verträglichen Nitride. Beispielsweise genannten werden Bornitrid, Siliciumnitird, Aluminiumnitrid.

Die mikromechanischen Verankerungsstellen dienen der Verbesserung der mechanischen Anbindung von Apatit an die Oberfläche. Beispielsweise kann der biomimetische Apatit in eine poröse oder auf einer aufgerauten Oberfläche hinein kristallisieren und so eine mechanische Verankerung des Apatits auf der Oberfläche bewirken.

Gegenstand der Erfindung ist ein Formteil zur Verwendung zur dentalen, prothetischen Versorgung bei Zahnverlust, insbesondere zur zellulären Anheftung von Zellen synonym Zelladhäsion, vorzugsweise zur Anheftung von prothetischen Formteilen über Hemidesmosome. Besonders bevorzugt ist die Verwendung zur Anheftung von Zellen, wie Epithelzellen oder Fibroblasten, an prothetische Formteile. Die zelluläre Anheftung von Zellen über Hemidesmosomen oder andere biologische Mechanismen an prothetische Formteile wird für die dauerhafte, biologische Integration der Implantate bei den Patienten angestrebt. Die dentale hemidesmosomale Anheftung von dentalen, prothetischen Formteilen ist für die Regeneration der Gingiva/des Zahnfleisches aufgrund der Epithelproliferation und deren Anheftung an das prothetische Formteil wichtig, um auf diese Weise eine dauerhafte biologische Verbindung zum Implantat auszubilden und die Randspalten zu minimieren. Hemidismosome sind Haftklomplexe, die die Adhäsion von Epithelzellen an einer extrazellulären Matrix vermitteln. Die Haftkomplexe der Hemidesmosomen weisen einen typischen Aufbau aus intrazellulärer Plaqueproteine, Intermediärfilamenten und transmembranen Kontaktproteinen auf.

Nach einer Implantation wird angestrebt, dass das peri-implantäre Gewebe biologisch an die Implantatoberfläche, d.h. die Oberfläche des prothetischen Formteils, angebunden werden kann. Diese Anbindung gelingt für übliche prothetische Formteile aufgrund von Abstossungsreaktionen des Körpers gegenüber den körperfremden Materialien nicht. Demgegenüber bietet der biomimetisch abgeschiedene Apatit eine ideale Basis zur zellulären Anheftung von Zellen, da der biomimetische Apatit den Epithelzellen oder Fibroplasten der Gingiva und dem marginalen, parodontosem Gewebe quasi "eigenes" Gewebe präsentiert. Der biomimetische Apatit, umfassend Hydroxylapatit, Fluorapatit und Gemische dieser, wird von den Zellen, insbesondere den Gingiva-Epithelzellen und/oder Fibroblasten, als "natürliche Zahnoberfläche" erkannt. Während bei Knochenersatzwerkstoffen auf Apatitbasis eine Auflösung und Remineralisation des Knocheersatzwerkstoffes (KEW) erwünscht ist, ist bei einer Implantatbeschichtung wichtig, dass die Zellen die Apatitoberfläche als körperverwandt erkennen und nicht auflösen, da sonst wieder Implantat, hier das Titan, freigelegt würde. Fluorapatit ist dem humanen Schmelz ähnlich genug, um als körperverwandt erkannt zu werden, wird aber nicht aufgelöst.

Erfindungsgemäss ist der biomimetische Apatit, umfassend Hydroxylapatit, Fluorapatit und Gemische dieser, kristallin. Erhalten wird nadelförmiger, biomimetischer Apatit, wobei zwischen den Schichten des Apatits oder in Kavitäten organische Verbindungen des Gelbildners, vorzugsweise der Gelatine, eingelagert sind. Der Gehalt an Gelbildner im Apatit kann von 0,001 bis 10 Gew.-%, insbesondere 0.5 bis 5 Gew.-% in Bezug auf die Gesamtzusammensetzung an biomimetischem Apatit betragen. Der biomimetische Apatit ist dem Apatit des Zahnschmelzes ähnlich.

Durch die erfindungsgemässen, mit biomimetisch abgeschiedenem Apatit beschichteten, Formteile ist die Anlagerung periimplantären Weichgewebes aufgrund der ungestörten zellulären Anheftung über Hemidesmosomen oder andere biologische Mechanismen von Gingiva-Epithelzellen, Fibroblasten oder anderen Zellen möglich, so dass der Übergang Formteil Zahnfleisch verschlossen werden kann. Erfindungsgemäss kann auf diese Weise eine biologische Barriere durch die zelluläre Anheftung geschaffen werden, die die Risiken einer periimplantären Entzündung durch Bakterien deutlich vermindert.

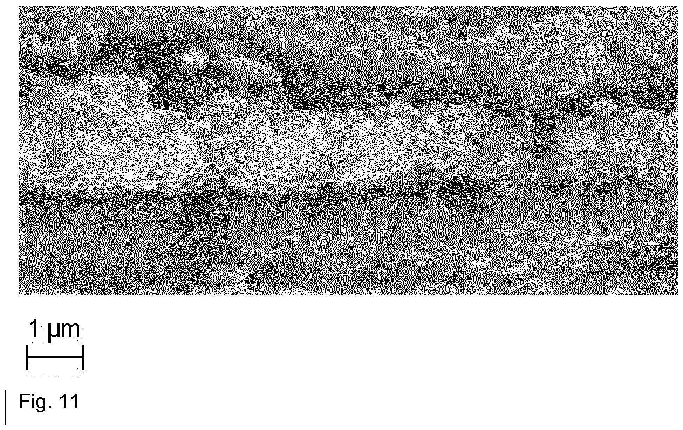

Erfindungsgemäss kann durch Anwendung des erfindungsgemässen Verfahrens die Morphologie und die Wachstumsorientierung des Apatits entsprechend dem des natürlichen Zahnschmelzes erzeugt werden. Der biomimetische Apatit zeigt vorzugsweise eine Parallelorientierung der nadeligen Kristallite wie sie beim Zahnschmelz oberflächennah zu beobachten ist. Die Kristallitgrösse und Orientierung von biomimetisch abgeschiedenem Apatit ist exemplarisch in Figur 11 wiedergegeben. Grundsätzlich kann die Kristallitgrösse variieren, wobei die größeren Kristallite einen Durchmesser von etwa 100-250 nm und eine Länge von etwa 500-1000 nm aufweisen. Generell kann die Kristallitgrösse sowohl nach unten und nach oben je nach Kristallisationsbedingungen abweichen. Nach einer Alternative der Erfindung kann es bevorzugt sein, wenn die biomimetischen Apatitoberflächen, die auf dem prothetischen Formteilen aufgewachsen sind, poliert werden, um die Oberflächenrauigkeit der Apatitoberfläche (zur epithelialen Manschette hin) zu reduzieren. Die polierte Apatitoberfläche weist vorzugsweise eine geringere Rauigkeit von +/- 0,005 bis 10 µm, insbesondere von 0,05 bis 5 µm,besonders bevorzugt von 0,005 bis 2 µm auf, während eine unpolierte Apatitoberfläche eine Rauigkeit von größer 10,5 µm aufweisen kann. So wurde eine Probe mit einer Differenz von etwa 35 bis 40 µm in der Höhe der aufgewachsenen Apatitschicht vermessen. Die geschieht, um die bakterielle Adhärenz auf der Apatitoberfläche zu minimieren.

Nach einer besonders bevorzugten Ausführungsform der Erfindung umfasst biomimetischer Apatit einen Gesamtgehalt an C,H,N-Atomen, die in elektrolytisch abgeschiedenem Apatit oder gesputtertem Apatit nicht auftreten. Vorzugsweise liegt der Gesamtgehalt an C,H,N-Atomen im Bereich von 0,01 Gew.-% bis 10 Gew.-%, insbesondere von 0,05 bis 5,0 Gew.-%, besonders bevorzugt um 2,3 Gew.-% mit einer Varianz von plus/minus 0,5 Gew.-%. Der biomimetische Apatit kann auch als biomineralisierter Apatit aus Kollagen abgeschieden werden und kann dann Bestandteile von Kollagen, denaturiertem Kollagen, Aminosäurederivaten oder Proteinen umfassen. Vorzugsweise umfasst der biomimetische Apatit zwischen den Apatitschichten und/oder in Kavitäten Aminosäuren, Aminosäurederivate, Proteine, denaturiertes Kollagen. Ebenso kann der Apatit, insbesondere zwischen den Apatitschichten und/oder in Kavitäten, Bestandteile von Kollagen, denaturiertem Kollagen, Gelatine, Proteinketten und/oder Gelatine-Glycerin-Gel aufweisen. Vorzugsweise umfasst der biomimetische Apatit von 0,001 Gew.-% bis 15 Gew.-% Kollagen, denaturiertes Kollagen, Proteine und/oder Aminosäurederivate, insbesondere von 0,01 Gew.-% bis 10 Gew.-%, bevorzugt von 0,1 bis 5 Gew.-%, besonders bevorzugt von 0,5 bis 5 Gew.-% in Bezug auf die Gesamtzusammensetzung. Nach einer besonders bevorzugten Alternative umfasst das Formteil biomimetischen Apatit oder Fluorapatit mit einem Gehalt an Kohlenstoff und optional einem Gehalt an Stickstoff. Vorzugsweise liegt der Gehalt an Kohlenstoff im Bereich von 0,25 bis 2,5 Gew.-%, vorzugsweise von 0,25 bis 1,0 Gew.-% und optional der Gehalt an Stickstoff im Bereich von 0,09 bis 0,9 Gew.-%. Nachgewiesen werden kann der Gehalt an denaturiertem Kollagen bzw. der Gelatine über die Bestimmung des Kohlenstoff-und/oder Stickstoffgehaltes bspw. mittels Elementaranalyse. Es wird angenommen, dass die Einlagerung des Gelbildners, insbesondere der Gelatine, zwischen den Apatitschichten oder in Kavitäten des Apatits erfolgt. So konnten mit einer DTA-MS Analyse Fragmente mit MM-Peaks mit einem Molekulargewicht von 18 und 44 (CO₂ und Wasser), die Degradationsprodukte von Gelatine nachgewiesen werden. Ein Erhitzen der Schichten bis 1000°C kann zu einer Schwarzfärbung führen, da Gelatine zu Kohlenstoff verbrannt wird. Der Nachweis des kristallinen Apatits oder kristallinen Fluorapatits kann mittels XRD erfolgen.

Das prothetische Formteil umfasst, vorzugsweise in einem Bereich der später mit Epithelzellen, Fibroblasten, anderen Zellen, dem Zahnfleisch, der Gingiva - wie beispielsweise Gingiva-Epithelzellen oder dem Bereich der epithelialen Manschette (Saumepithel) in Kontakt kommt, eine Apatitschicht. Diese Apatitschicht kann eine oder eine Vielzahl an Apatitschichten, wie 1, 2, 3 bis 50 bis unendlich viele Schichten, umfassen. Je nach Herstellverfahren wird eine Apatitschicht oder eine Vielzahl an Apatitschichten auf den Bereich des Formteils aufgebracht. Erfindungsgemäss wird das prothetische Formteil im Bereich der Apatitschicht von der Gingiva als epitheliale Manschette (Saumepithel) umschlossen, so dass die Eintrittsstelle des Implantates bzw. prothetischen Formteils abgeschlossen wird.

Nach einer besonders bevorzugten Ausführungsform der Erfindung beträgt die Schichtdicke des biomimetischen Apatits umfassend mindestens eine oder mehrere Apatitschichten von 100 nm bis 1 mm, insbesondere von 500 nm bis 800 µm, bevorzugt sind 1 µm bis 500 µm, besonders bevorzugt sind Schichtdicken von 10 µm bis 500 µm. Alternative Schichtdicken aus biomimetischem Apatit liegen im Bereich von 10 µm bis 100 µm, von 20 µm bis 200 µm, von 50 µm bis 100 µm, von 100 µm bis 200 µm, von 100 µm bis 400 µm oder von 1 µm bis 50 µm. Weiter bevorzugt sind mittlere Schichtdicken des biomimetischen Apatits im Bereich von 5 µm bis 200 µm, bevorzugt von 10 µm bis 200 µm.

Die erfindungsgemässen prothetischen Formteile umfassen generell alle prothetischen Formteile, wie medizinische, orthopädische, kieferorthopädische, dentale Formteile sowie prothetische Formteile zur Verankerungen, wie Implantate, Schrauben, Nägel, chirurgische Platten, als auch prothetische Formteile als Knochenersatz, wie orthopädische Prothesen, Gelenkprothesen, wie Gelenk-Endoprothesen, Revisions-Total-Gelenk-Endoprothesen, Knochenprothesen, Wirbelkörper, Dornfortsatz oder Teile davon. Bevorzugte prothetische Formteile umfassen enossale Implantate, dentale, enossale (intraossär) Implantate, Verbindungselement, Aufbauelement, dentale Hülse, Abutment, Superstruktur/Suprastruktur, Teil einer dentalen Prothese, dentale Totalprothese, orthopädische Prothese oder Teile davon, künstlicher Zahn, Veneer, Inlay, Onlay, dentale Trägerstruktur, Brücke, Krone, Unterfütterung, Prothesensattel und/oder Spacer. Besonders bevorzugt ist als Formteil ein enossales Implantat; insbesondere dentales, enossales Zahnimplantat, ein Implantatpfosten; ein Verbindungselement für ein Implantat, ein Aufbauelement, wie ein Abutment für ein dentales Implantat sowie chirurgische Implantate und alle Teile, die einem Implantat oder einer Superkonstruktion zugeordnet werden. Dem Fachmann ist klar, dass erfindungsgemäss als prothetische Formteile alle geeigneten Formteile aus jeglichen physiologisch geeigneten Materialien mit einer biomimetischen Apatitschicht versehen werden können.

Generell können die prothetischen Formteile aus allen geeigneten physiologisch verträglichen Materialien hergestellt sein. Besonders bevorzugt kann das Formteil einen metallischen Werkstoff, eine Legierung, eine dentale Legierung, Keramik, einen Hybridwerkstoff umfassen. Bevorzugt kann das Formteil aus Titan, Titanlegierung, Titanoxid, Kobalt-Chrom-Legierung, CoCrMo-Legierung, Gold, dentaler Keramik, Zirkonoxid, insbesondere ZrO₂, Lithiumdisilikat, Polymer, Polymergemisch, dentalem Prothesenkunststoff gebildet sein.

Ebenso ist es bevorzugt, wenn das Formteil neben den biomimetisch eingeschlossenen oder durch die Fällung oder die Kristallisation von Apatit in den Kristallen oder zwischen den Kristallen eingeschlossenen im Wesentlichen biologischen Komponenten, wie Proteinen, Kollagen, denaturiertes Kollagen, auch Aminosäuren umfasst. Gegenstand der Erfindung ist daher auch ein Formteil umfassend eine Schicht von biomimetischem Apatit, welches umfasst Aminosäuren, Aminosäurederivate, Proteine, denaturiertes Kollagen und/oder Gelatine-Glycerin-Gel.

Ferner kann das Formteil zumindest bereichsweise an seiner Oberfläche Mikroretentionen als mikromechanische Verankerungsstellen in der Oberflächentopographie aufweisen. Diese Mikroretentionen dienen als mechanische Verbindungsstellen und umfassen vorzugsweise eine poröse, rissige und/oder raue Oberflächentopographie. Besonders bevorzugt ist die Oberfläche zumindest in diesen Bereichen oder auch bis zur gesamten Oberfläche porös. Diese Oberflächentopographie bzw. die mechanischen Verbindungstellen können erzeugt werden, indem mindestens ein Bereich der Oberfläche des Formteils mechanisch, insbesondere mittels Sandstrahlen bspw. mit Korund, Haselnüssen oder anderen gängigen Medien behandelt wird. Ebenso kann die Oberfläche chemisch, wie durch Ätzen mittels Säure oder Alkalie, elektrochemisch, beispielsweise in einem elektrolytischen Verfahren und/oder in einem Plasmaverfahren behandelt und/oder aktiviert werden. Gleichfalls kann die Oberfläche in einem Verfahren, das die vorgenannten Verfahren kombiniert, behandelt und/oder aktiviert werden, wobei sich durch die Behandlung mikromechanische Verankerungsstellen ausbilden. Besonders bevorzugt ist die Ausbildung mikromechanischer Verankerungsstellen, die zusätzlich chemisch aktiviert werden. Dies kann beispielsweise die Bildung einer porösen und/oder rauen Oberfläche umfassen, die anschliessend, unter Ausbildung von chemischen Verankerungsstellen, chemisch geätzt wird. Diese chemischen Verankerungsstellen können eine biomimetische Kristallisation des Apatits sowie dessen feste Bindung an das Formteil begünstigen.

Nach einer weiteren Alternative können die Formteile auch in einem aufbauenden oder abtragenden Verfahren so hergestellt werden, dass die Oberfläche mikromechanische Verankerungsstellen aufweist. Ein mögliches aufbauendes Verfahren umfasst das Lasersintern, ein abtragendes Verfahren kann fräsend optional kombiniert mit einem elektrolytischen Verfahren umfassen, um eine Oberfläche mit Verankerungsstellen, wie vorzugsweise eine poröse Oberfläche, zu erhalten. Typischerweise kann Titandioxid oder eine andere metallische Legierung elektrochemisch unter Bildung von Gas oder einer Zersetzung der Legierung behandelt werden. Die Oberfläche umfassend mindestens bereichsweise mikromechanische Verankerungsstellen umfasst vorzugsweise eine raue und/oder poröse Oberflächentopografie. Die Oberfläche umfassend Verankerungsstellen kann daher eine andere chemische Beschaffenheit als der verbleibende Teil des prothetischen Formteils aufweisen. Ein Beispiel dafür kann eine Titanlegierung sein, die bereichsweise eine poröse, oxydische Oberfläche aufweist. Als porös gilt vorzugsweise eine Oberfläche, wenn die Oberfläche Hinterschneidungen und/oder Poren aufweist.

Der biomimetische Apatit wird nach der Theorie der Erfindung, ohne an diese gebunden zu sein, an den Verankerungsstellen der Oberfläche, vorzugsweise in und/oder an einer porösen Oberfläche nach dem erfindungsgemässen Verfahren abgeschieden, so dass der biomimetische Apatit mit den Verankerungsstellen der Oberfläche, insbesondere der porösen Oberfläche, fest verwachsen ist.

Ferner ist Gegenstand der Erfindung ein prothetisches Formteil oder einen Teil dessen, mit
a) einer Oberfläche die mindestens bereichsweise in dem Bereich, der später im Bereich des Zahnfleisches, d.h. an der Durchtrittstelle vom Mund in den Kiefer, angeordnet ist mikromechanische Verankerungsstellen aufweist, insbesondere Mikroretentionen in der Oberflächentopographie und/oder chemische Verankerungsstellen,
b) einer Oberfläche die mindestens bereichsweise in dem Bereich, der später im Bereich von Epithelzellen, Fibroblasten, dem Zahnfleisch, der Gingiva, insbesondere der Gingiva-Epithelzellen, oder dem Bereich der epithelialen Manschette (Saumepithel) angeordnet ist mikromechanische Verankerungsstellen aufweist, wobei das prothetische Formteil ausgewählt ist aus Verbindungselement, Aufbauelement, Abutment, Implantat, oberem, äusseren prothetischen Aufbau sowie den weiteren gennannten prothetischen Teilen.

Die biomimetisch abgeschiedene Apatitschicht auf dem prothetischen Formteil kann nach einer Alternative der Erfindung oberflächlich geglättet und/oder poliert werden. Dem Fachmann sind übliche Massnahmen zum Glätten und/oder Polieren im dentalen Bereich bekannt.

Ebenso Gegenstand der Erfindung ist ein Verfahren zur Abscheidung von biomimetischem Apatit auf einem prothetischen Formteil, insbesondere einem dentalen prothetischen Formteil, ausgewählt aus Fluorapatit, Hydroxylapatit oder deren Gemischen, umfassend die Schritte:
(i.a) Bereitstellen eines prothetischen Formteils, wobei mindestens ein Bereich der Oberfläche des Formteils mikromechanische Verankerungsstellen aufweist, vorzugweise sind die mikromechanischen Verankerungsstellen erhältlich durch mechanische, chemische, elektrochemische Behandlung, Plasmabehandlung, Ätzen, Abscheiden oder einer Kombination der genannten Behandlungen oder
(i.b) Behandeln zumindest eines Bereiches der Oberfläche eines prothetischen Formteils, wobei das Behandeln vorzugsweise umfasst eine mechanische, chemische, elektrochemische Behandlung, Plasmabehandlung, Ätzen, Abscheiden oder Kombinationen der der vorgenannten Behandlungen, optional gefolgt von einem Reinigen, insbesondere Waschen der Oberfläche, und Erhalten von mikromechanischen Verankerungsstellen, insbesondere mikromechanischen und optional chemischen Verankerungsstellen, und optional
(ii) Behandeln zumindest eines Bereiches der Oberfläche eines prothetischen Formteils mit einer Vorbehandlungszusammensetzung mit einem definierten pH-Wert, insbesondere pH 8 bis 10,
(iii) In-Kontaktbringen mindestens dieses Bereiches, insbesondere der behandelten Oberfläche, des prothetischen Formteils umfassend mikromechanische Verankerungsstellen mit einer Phosphat-Ionen enthaltenden Zusammensetzung, welche einen Gelbildner umfasst, vorzugsweise Gelatine, wobei eine Gelschicht ausgebildet wird, vorzugsweise zumindest erfolgt teilweises Bedecken der Oberfläche mit dem Gel, wobei die bedeckten Bereiche der Oberfläche im Wesentlichen vollständig und gleichmässig mit einer Gelschicht bedeckt werden, insbesondere mit einer Gelschicht von 0,001 mm bis 10 mm, bevorzugt von etwa 1 mm bis 5 mm,
(iv) optional Auftragen einer weiteren Schicht unter Ausbildung einer weiteren Gelschicht, insbesondere durch In-Kontaktbringen der ersten Gelschicht mit einer weiteren Zusammensetzung, die einen Gelbildner umfasst, der insbesondere frei von Phosphat-Ionen, Calcium und/oder Fluorid-Ionen ist, vorzugsweise weist die Gelschicht eine Dicke von 0,001 mm bis 10 mm auf, bevorzugt von etwa 1 mm bis 5 mm, und
(v) In-Kontaktbringen der ersten Gelschicht oder der weiteren Schicht mit einer Calcium-Ionen enthaltenden Zusammensetzung, wobei eine Gelschicht ausgebildet wird,
(vi) Abscheidung von biomimetischem Apatit ausgewählt aus Fluorapatit, Hydroxylapatit oder deren Gemischen, insbesondere kristallinem biomimetischen Apatit, auf der Oberfläche des prothetischen Formteils. Ferner ist Gegenstand der Erfindung ein prothetisches Formteil erhältlich nach dem erfindungsgemässen Verfahren. Die Einwirkzeit zur Abscheidung des Apatits kann von 1 bis 48 Stunden dauern, bevorzugt ist eine ein- bis mehrfache Anwendung von je 1 bis 10 Stunden. Dabei ist es besonders bevorzugt, wenn das Verfahren bei einer Temperatur von 20 bis 40 °C, vorzugsweise von 30 bis 39°C durchgeführt wird.

Das Inkontaktbringen mit der Gelschicht kann erfolgen, indem beispielsweise Gelfäden oder Gelstreifen um ein Implantat gewickelt werden und eine Vielzahl von Gelfäden nebeneinander eine Gelschicht ausbilden. Die Fäden können beispielsweise analog einer Garnrolle um die prothetischen Formteile gewickelt werden. Ebenso ist ein Aufsprühen, Auftragen mit dem Pinsel, Auftragen aus einer Düse oder Aufdrucken der Gelschichten möglich.

Vorzugsweise erfolgt eine Abscheidung von biomimetischem Apatit mit einer Schichtdicke von mindestens 1 µm, vorzugsweise von 1 µm bis 500 µm, bevorzugt 2 µm bis 200 µm, besonders bevorzugt von 5 µm bis 100 µm.

Die Behandlung der Oberfläche eines prothetischen Formteils umfasst besonders bevorzugt eine zusätzliche Behandlung der Oberfläche mit mikromechanischen Verankerungsstellen mit einer Vorbehandlungszusammensetzung. Diese Zusammensetzung kann auf die Oberfläche aufgetragen werden und kann auf diese einwirken. Auf die Vorbehandlungszusammensetzung kann die Phosphat-Ionen enthaltende Zusammensetzung aufgetragen werden. Alternativ kann die Vorbehandlungszusammensetzung nach einer Einwirkzeit entfernt werden.

Die Verfügbarkeit von Phosphat in der Phosphat-Ionen enthaltenden Zusammensetzung (erstes Gel) kann bevorzugt durch die Zugabe von Aminosäuren mit zusätzlichen basischen Gruppen erhöht werden. Zur Erhöhung der Löslichkeit sind weiterhin alle Substanzen geeignet, die über Bindungsstellen für Calcium- und Phosphat-Ionen verfügen ohne diese auszufällen oder toxisch auf den menschlichen Organismus wirken. Darunter fallen beispielsweise Vitamine (z.B. Ascorbinsäure), Oligopeptide, Carbonsäuren, insbesondere Fruchtsäuren, wie Äpfelsäure, Zitronensäure, Milchsäure oder Brenztraubensäure oder Komplexbildner wie EDTA. Erfindungsgemäss wird vorgeschlagen, 1-3 unterschiedliche Gelschichten zu kombinieren, wobei die Reihenfolge festgelegt ist.

Es wird ein Zwei-Gel-Schichten-Verfahren vorgeschlagen, in dem das Deck-Gel einen induzierenden Effekt auf die Mineralisation hat. Die Verwendung dünner, vorkonfektionierter Gelfilme ist in einem industriellen Verfahren wirtschaftlicher. Diese Gelfilme können beispielsweise auf die prothetischen Formteile aufgerollt werden. Alternativ kann das prothetische Formteil über die Zusammensetzung geführt werden, wobei die jeweilige Zusammensetzung auf einem definierten Bereich der Oberfläche des Formteils oder einer bereits aufgetragenen Zusammensetzung haften bleibt. Vorzugsweise wird das Formteil mit einer erwärmten Zusammensetzung, d.h. verflüssigten Zusammensetzung, in Kontakt gebracht. Die Zusammensetzung kann dann auf dem Formteil durch Abkühlen verfestigt werden. Alternativ kann das Formteil mit einer erwärmten flüssigen Gellösung besprüht werden. Ebenfalls bevorzugt ist ein Umwickeln der Formteile mit einem vorkonfektionierten Gelfaden oder Gelstreifen.

Die Schichtdicke der Phosphat-Gelschicht oder Calciumposphat-Gelschicht beträgt vorzugsweise von 50 µm bis 1 cm, insbesondere von 100 µm bis 1000 µm, bevorzugt 150 µm bis 500 µm. Die Konzentration der Phosphat-Ionen Zusammensetzung beträgt von 0,01 mol/l bis 2 mol/l, bevorzugt von 0,08 mol/l bis 0,3 mol/l. Die Fluorid-Ionen Konzentration beträgt von 0 mol/l bis 0,3 mol/l, bevorzugt von 0,0001 mol/l bis 0,05 mol/l. Die Konzentration der Calcium-Ionen beträgt vorzugsweise von 0,0001 mol/l bis 0,1 mol/l in der Zusammensetzung. Die Schichtdicke des Calciumgels beträgt vorzugsweise von 50 µm bis 5 mm, vorzugsweise von 300 µm bis 2500 µm, bevorzugt von 300 µm bis 1500 µm.

Überraschenderweise wurde gefunden, dass eine calciumfreie, alkalische Fluorid-Ionen enthaltende Vorbehandlungszusammensetzung einen wachstumsbeschleunigenden Effekt auf die Bildung der Apatitschicht hat. Der Zusammensetzung kann Gelatine zugefügt werden, um ihr eine gelige Konsistenz zu verleihen und die Haftung an der Oberfläche zu verbessern.

Die Behandlung zumindest eines Bereiches der Oberfläche eines prothetischen Formteils kann eine mechanische, chemische, elektrochemische Behandlung und/oder eine Behandlung mittels eines Plasmaverfahrens umfassen, um vorzugsweise die mikromechanischen Verankerungsstellen zu erzeugen. An diese Behandlung kann sich eine weitere Behandlung mit einer sauren oder vorzugsweise alkalischen Vorbehandlungszusammensetzung anschliessend, um die erzeugten mikromechanischen Verankerungsstellen chemisch zu aktivieren und/oder zu säubern.

Erfindungsgemäss werden die mikromechanischen Verankerungsstellen benötigt, um einerseits eine vorzugsweise gleichmässige Verteilung der Vorbehandlungszusammensetzung auf der Oberfläche des Formteils zu ermöglichen und/oder andererseits, um eine feste und dauerhafte mechanische Verankerung des biomimetisch abgeschiedenen Apatits mit dem Formteil, durch ein Einkristallisieren des Apatits in die poröse Oberfläche des Formteils zu ermöglichen. Die Oberfläche des Formteils weist mikromechanische Verankerungsstellen auf, die vorzugsweise in Form einer porösen Oberfläche vorliegen. Zudem können kanalartige Strukturen als Verankerungsstellen in der Oberfläche ausgebildet sein. Die Porendurchmesser der porösen Oberfläche des prothetischen Formteils, insbesondere von Implantate im infrakrestalen Bereich, liegen vorzugsweise im Bereich von etwa 0,5 bis 150 µm, besonders bevorzugt liegt der Porendurchmesser nach einer Alternative bei 50 bis 120 µm, vorzugsweise bei 65 bis 105 µm. Nach einer weiteren Alternative ist es bevorzugt, wenn der Porendurchmesser der prothestischen Formteile zur Abscheidung von biomimetischem Apatit ein Vielfaches der Kristallitdurchmesser beträgt. Dies erleichtert das Einkristalliesieren des biomimetischen Apatits in die poröse Oberfläche und bedingt somit eine feste mechanische Verankerung. Daher kann es ebenso ausreichend und bevorzugt sein, wenn die Porendurchmesser etwas kleiner im Bereich von 0,2 µm bis 70 µm liegen, vorzugsweise 0,2 µm bis 50 µm, besonders bevorzugt von 0,2 µm bis 20 µm, insbesondere im Bereich von 0,5 µm bis 10 µm oder auch von 0,5 µm bis 5 µm. Weiter bevorzugt ist ein Bereich von 1 µm bis 10 µm. Die mittlere Rauheit der Oberfläche des Bereiches mit mikromechanischen Verankerungsstellen kann im Bereich von Rₐ (mittlere Rauheit, Linienprofil 2D) von Rₐ größer gleich 0,75 bis 4 µm liegen, insbesondere von 1,0 bis 4,0 µm, von 1,0 bis 3,0 µm. Mittels CNC-Fräsmaschinen hergestellte Formteile weisen einen Ra Wert von um 0,29 µm auf. Die mittlere Rauigkeit Sₐ (Flächenprofil, 3D) der Oberfläche mit mikromechanischen Verankerungsstellen beträgt vorzugsweise bei den Oberflächen mit mikromechanischen Verankerungspunkten Sₐ größer gleiche 1,2 µm bis 4,0 µm, insbesondere größer gleiche 1,25 µm bis 3,0 µm. Die mittlere Flächenrauigkeit einer mittels CNC-Fräsmaschine hergestellten Oberfläche liegt bei unter 0,75 µm.

Die erfindungsgemässe Vorbehandlungszusammensetzung aktiviert vorzugsweise die Oberfläche umfassend mikromechanische Verankerungspunkte oder ist, je nach Material der Oberfläche, in der Lage durch eine Ätzung die mikromechanischen Verankerungspunkte zu bilden, so dass die biomimetische Abscheidung von Apatit und/oder Fluorapatit verbessert wird. Diese Aktivierung kann die Bildung von oxydischen Bereichen, die Bildung von Hydroxy-Gruppen und/oder eine Säuberung der Oberfläche umfassen.

Die alkalische Vorbehandlungszusammensetzung kann umfassen eine alkalische Vorbehandlungszusammensetzung, insbesondere mit einem pH-Wert von 8 bis 14, insbesondere 9 bis 14, vorzugsweise einem pH-Wert um 14. Besonders bevorzugt ist eine Natriumfluoridlösung oder ein Natriumfluoridgel, wie umfassend 0,01 mol bis 3,0 mol NaF. Bevorzugt umfasst das Vorbehandlungszusammensetzung um 0,1 mol bis 2,0 mol NaF, besonders bevorzugt ist ein 0.5 molare Natriumfluoridlösung die auf pH 14 eingestellt ist und optional einen Gehalt an Gelatine aufweist, vorzugsweise beträgt der Gehalt an Gelatine von 1 bis 20 Gew.-%, insbesondere 5 bis 10 Gew.-%, weiter bevorzugt sind etwa 7.5 Gew.-% Gelatine. Die alkalische Vorbehandlungszusammensetzung verbleibt auf der Oberfläche des prothetischen Formteils. Auf die Vorbehandlungszusammensetzung werden anschliessend zunächst das Phosphat-Ionen enthaltende Gel und nachfolgend das Calcium-Ionen enthaltende Gel aufgebracht.

Erfindungsgemäss wird in dem Verfahren der Erfindung zudem die Vorbehandlungszusammensetzung, insbesondere zur Aktivierung der Oberfläche mit mikromechanischen Verankerungspunkten oder zur Bildung der mikromechanischen Verankerungspunkte eingesetzt, wobei die Vorbehandlungszusammensetzung einen pH-Wert von 8 bis 14 aufweist, insbesondere pH 8 bis 10 oder auch pH 12 bis 14, und vorzugsweise Fluorid-Ionen umfasst, wie NaF, NH₄F oder andere lösliche Aminfluoride. Zur Einstellung des pH-Wertes können Alkalihydroxide, wie NaOH, KOH oder Erdalkalihydroxide, wie Ca(OH)₂ etc. verwendet werden.

Durch die Verwendung der alkalischen Vorbehandlungszusammensetzung bilden sich reproduzierbar sehr dünne Filme auf der Oberfläche des prothetischen Formteils aus, welche die initiale Mineralisation besonders begünstigen. Je nach lonengehalt der Phosphat- und/oder Calcium-Ionen enthaltenden Zusammensetzung, d.h. des ersten oder zweiten Gels, kann die Vorbehandlungszusammensetzung von 0,5 mol/l bis 3 mol/l Calcium-Ionen, oder von 0,0 mol/l bis 1 mol/l Phosphat-Ionen, insbesondere 0,001 mol/l und/oder von 0 mol/l bis 1 mol/l Fluorid-Ionen enthalten. Der pH-Wert kann von 8 bis 14 betragen, insbesondere pH 8 bis 10.

Ebenfalls bevorzugt ist eine alkalische Vorbehandlungszusammensetzung mit einer 0.5 molaren, wässrigen Natriumfluoridzusammensetzung, die auf pH 14 eingestellt ist und optional etwa von 0,1 bis 7,5 Gew.-% Gelatine umfasst. Gleichfalls kann eine alkalische Vorbehandlungszusammensetzung mit einer 0,5 bis 3,0 molaren, vorzugsweise 1,0 bis 3,0 molaren, wässrigen Calcium-Ionen enthaltenden Zusammensetzung, die auf pH 14 eingestellt ist, verwendet werden. Die prothetischen Formteile werden bereichsweise an der Oberfläche mit dieser Zusammensetzung eingestrichen und die Feuchtigkeit wird im Anschluss danach verblasen.

Nach einer bevorzugten Verfahrensalternative wird in dem erfindungsgemässen Verfahren eine (a) Phosphat-Ionen enthaltende Zusammensetzung eingesetzt, die umfasst,
(a.1) mindestens einen Gelbildner,
(a.2) wasserlösliche Phosphate oder zu wasserlöslichen Phosphat-Ionen hydrolysierbare Phosphate, insbesondere Phosphat- oder Hydrogenphosphat-Ionen,
(a.3) optional Fluorid, (a.3.1) optional eine oder mehrere Aminosäuren,
(a.4) optional eine Carbonsäure oder ein Puffersystem von pH 4 bis 7,
(a.5) optional Glycerin. Diese Zusammensetzung kann auch als erstes Gel bezeichnet werden.

Ferner wird nach einer bevorzugten Verfahrensalternative in dem erfindungsgemässen Verfahren eine (b) Calcium-Ionen enthaltende Zusammensetzung eingesetzt, die umfasst,
(b.1) mindestens einen Gelbildner,
(b.2) Calcium-Ionen
(b.3) optional Glycerin. Diese Zusammensetzung kann auch als zweites Gel bezeichnet werden.

Die Zusammensetzungen weisen vorzugsweise einen pH-Wert von 2 bis 9 auf, bevorzugt von 3 bis 8, besonders bevorzugt von 4 bis 7, weiter bevorzugt von 4 bis 6.

Die erfindungsgemässen Zusammensetzungen können jeweils unabhängig Aminosäuren, Derivate von Aminosäuren, Proteine oder denaturiertes Kollagen umfassen.

Die Gelbilder können ausgewählt werden aus denaturiertem Kollagen, Gelatine, Gelatine-Glycerin-Gel, Hydrokolloiden, Polypeptiden, Proteinhydrolysaten, Polysacchariden, Polyacrylaten oder Mischungen umfassend mindestens zwei der genannten Gelbildner. Vorzugsweise umfassen die Zusammensetzungen jeweils unabhängig Gelatine und ein Polyol, vorzugsweise Glycerin, deren Addukte und/oder deren Umsetzungsprodukte.

Die erfindungsgemässen Zusammensetzungen, insbesondere die Phosphat-Ionen und/oder Calcium-Ionen enthaltende Zusammensetzung, die Vorbehandlungszusammensetzung, umfassen vorzugsweise jeweils unabhängig einen Gehalt an Wasser. Der Gehalt an Wasser kann variieren in der Phosphat-Ionen enthaltenden Zusammensetzung, insbesondere im Gel, von etwa 45 bis 55 Gew.-% und in der Calcium-Ionen enthaltenden Zusammensetzung, insbesondere im Gel, von etwa 30 bis 40 Gew.-%, jeweils in Bezug auf die Gesamtzusammensetzung.

Das Behandeln der Oberfläche des Formteils umfasst eine mechanische, chemische, elektrochemische Behandlung und/oder eine Behandlung in einem Plasmaverfahren oder eine Kombination der Verfahren, unter Ausbildung mindestens eines Bereiches der Oberfläche des prothetischen Formteils mit mikromechanischen Verankerungsstellen. Die Oberflächenbehandlung bewirkt eine Vergrösserung der Oberfläche, insbesondere wird eine poröse Oberfläche ausgebildet. Die so behandelte Oberfläche ist mikroretentiv, so dass der biomimetische Apatit dauerhaft mit der Oberflächenstruktur verwachsen kann. Ferner kann in dem erfindungsgemässen Verfahren die Oberfläche des Formteils zusätzlich mechanisch, chemisch, elektrochemisch und/oder in einem Plasmaverfahren oder durch eine Kombination der Verfahren aktiviert werden.

Ebenso Gegenstand der Erfindung ist die Verwendung von biomimetischem Apatit zur mindestens bereichsweisen Beschichtung prothetischer Formteile. Als biomimetischer Apatit wird erfindungsgemäss aus Gelatine oder Kollagen abgeschiedenes Apatit verstanden, das Bestandteile von Gelatine, Kollagen, denaturiertem Kollagen, Aminosäurederivaten oder Proteinen umfasst. Vorzugsweise umfasst der biomimetische Apatit von 0,001 Gew.-% bis 15 Gew.-% Kollagen, denaturiertes Kollagen, Proteine und/oder Aminosäurederivate, insbesondere von 0,01 Gew.-% bis 10 Gew.-%, bevorzugt von 0,1 bis 5 Gew.-%, besonders bevorzugt von 1 bis 10 Gew.-% in Bezug auf die Gesamtzusammensetzung. Der Nachweis der Abscheidung von biomimetischem Apatit kann über den Nachweis des Kohlenstoff- und/oder Stickstoffgehaltes erfolgen, den gesinterter Apatit nicht aufweist. Darüber hinaus bildet biomimetischer Apatit oder Fluorapatit eine makrokristalline flächige Kristallstruktur aus, in deren Poren und Hohlräumen Gelatine eingelagert ist, das die kristalline Phase aus einer feuchten Gelatine abgeschieden wurde. Demgegenüber weisen die aus Apatitpulvern über eine anschließende Sinterung hergestellten Apatite keine biomimetischen Makrostrukturen auf. Der gesinterte Apatit wird vom Körper nicht zur Anlagerung periimplantären Weichgewebes aufgrund der ungestörten zellulären Anheftung über Hemidesmosomen oder andere biologische Mechanismen von Gingiva-Epithelzellen, Fibroblasten oder anderen Zellen erkannt. Zudem kommt es unter anderem aufgrund der vorgenannten hohen Sintertemperaturen von um 1250 °C bei den Implantaten zu Verzügen der Geometrie des Implantatgrundgerüstes, so dass die Passgenauigkeit der Anschlussgeometrie der gesinterten Implantate nicht mehr stimmt.

Ferner ist Gegenstand der Erfindung die Verwendung von Phosphat-Ionen und Calcium-Ionen enthaltenden Zusammensetzungen, wie eines ersten Gels und eines zweiten Gels, oder von Formulierungen enthaltend diese Zusammensetzungen zur biomimetischen Abscheidung von Apatit auf einer Oberfläche eines prothetischen Formteils, wobei die Oberfläche mikromechanische Verankerungsstellen umfasst und/oder wobei die Oberfläche des Formteils vor der Abscheidung mechanisch, chemisch, elektrochemisch und/oder mittels einer Plasmabehandlung aktiviert wurde. Die aktivierte Oberfläche umfasst chemische Verankerungsstellen, wie funktionelle Gruppen, die beispielsweise oxydisch oder Hydroxyfunktionelle sein können.

Des Weiteren ist Gegenstand der Erfindung die Verwendung der vorgenannten Zusammensetzungen zur zumindest bereichsweisen Abscheidung von biomimetischem Apatit und/oder einer zumindest bereichsweisen, insbesondere in diesem Bereich, im Wesentlichen homogenen Abscheidung von Apatit. Als im Wesentlichen homogen gilt eine Abscheidung von Apatit, wenn der interessierende Bereich der Oberfläche des prothetischen Formteils zu mindestens 80 % mit einer Apatitschicht bedeckt ist, bevorzugt sind 90 %, besonders bevorzugt sind 90 bis 100%.

In einer nach der Erfindung besonders bevorzugten Ausführungsform umfasst die Phosphat-Ionen enthaltende Zusammensetzung: (i) wasserlösliche Phosphate oder zu wasserlöslichen Phosphat-Ionen hydrolysierbare Phosphate, insbesondere Na₂HPO₄, vorzugsweise liegt der Gehalt an Phosphat in der Zusammensetzung bei 1 bis 10 Gew.-%, vorzugsweise von 2 bis 8 Gew.-%, besonders bevorzugt von 5 bis 8 Gew.-%, (ii) einen Gehalt an Wasser oder ein Gemisch von Wasser und einem organischen Lösemittel, (iii) optional mindestens eine Carbonsäure, insbesondere eine Hydroxycarbonsäure, wie Milchsäure, und/oder ein Puffersystem, insbesondere liegt ein Puffersystem zur Einstellung des pH-Wertes im Bereich von 2 bis 8 vor, insbesondere von 3.5 bis 8, bevorzugt von 3.5 bis 6, besonders bevorzugt um 4,5 plus/minus 1,0, insbesondere plus/minus 0,5. Der Gehalt bezieht sich auf die eingewogene (HPO₄)²⁻ Konzentration.

In einer nach der Erfindung besonders bevorzugten Ausführungsform umfasst die Calcium-Ionen enthaltende Zusammensetzung: (i) Calcium-Ionen oder Calcium-Ionen freisetzende Verbindungen, insbesondere Calciumdichlorid oder Hydrate davon, vorzugsweise zusätzlich Calciumsulfat, Nanoapatit, Natriumcarbonat oder Calciumoxalat, vorzugsweise liegt der Gehalt an Calcium in der Zusammensetzung bei 1 bis 10 Gew.-%, vorzugsweise grösser gleich 1,5 bis 7,5 Gew.-%, (ii) optional Wasser oder ein Gemisch von Wasser und einem organischen Lösemittel, und (iii) optional mindestens eine Carbonsäure, wie eine Hydroxycarbonsäure bspw. Milchsäure, und/oder ein Puffersystem. Zur Herstellung der Puffer werden bevorzugt Fruchtsäuren und Alkali-Salze eingesetzt. Der Gehalt bezieht sich auf das Calcium (Ca²⁺). Der pH-Wert wird vorzugsweise auf 5,5 mit plus/mins 0,5 eingestellt.

Weiter ist es bevorzugt, wenn die Zusammensetzungen mindestens ein wasserlösliches Fluorid (F⁻), mit Fluorid-Ionen, oder eine Fluoride freisetzende Verbindung umfassen. Besonders bevorzugt weist die Phosphat-Ionen enthaltende Zusammensetzung als weitere Komponente (iv) mindestens ein wasserlösliches Fluorid oder eine Fluoride freisetzende Verbindung auf.

Nach einer bevorzugten Ausgestaltung der Erfindung umfasst das mindestens eine wasserlösliche Fluorid oder die mindestens eine Fluoride freisetzende Verbindung, (iv) mindestens eine unsubstituierte oder substituierte Alkyl-Gruppe aufweisende quaternäre mono- oder poly-Ammonium-Verbindung, vorzugsweise mit vier substituierten AlkylGruppen, wobei die mindestens eine substituierte Alkyl-Gruppe umfasst Hydroxyalkyl-, Carboxyalkyl-, Aminoalkyl-Gruppen mit 1 bis 25 C-Atomen oder organofunktionelle durch Heteroatome unterbrochene Gruppen mit bis zu 50 C-Atomen. Bevorzugte AmmoniumVerbindungen können 1 bis 20 quaternäre Ammonium-Funktionalitäten enthalten, vorzugsweise 1, 2, 3, 4, 5, 6, 7, 8 Ammonium-Funktionalitäten, bevorzugt wird Olaflur (N,N,N'-Tris(2-hydroxyethyl)-N'-octadecyl-1,3-diaminopropandihydrofluorid) als wasserlösliches Fluorid eingesetzt. Gleichfalls bevorzugt sind Aminfluoride, wie Olaflur, Decaflur, Ethanolamin-Hydrofluorid, eine Fluoride freisetzende organofunktionelle Amino-Verbindung oder ein Fluoride freisetzendes Antiseptikum auf Basis von organofunktionellen AminoVerbindungen, wie insbesondere Fluoride von *N*-Octyl-1-[10-(4-octyliminopyridin-1-yl)decyl]pyridin-4-imin, Cetylpyridiniumfluorid, oder wasserlösliche anorganische Fluoride, wie Alkalifluoride, Natriumfluorid, Kaliumfluorid, Zinnfluorid, Ammoniumfluorid, oder Fluoride freisetzende anorganische Fluoride, wie Zinkfluorid, Zinkhydroxyfluorid.

Als erfindungsgemässer Gelbildner kann vorzugsweise mindestens ein Gelbildner, ausgewählt aus Gelatine, denaturiertem Kollagen, Hydrokolloiden, Polypeptiden, Proteinhydrolysaten, synthetische Polyaminosäuren, Polysacchariden oder Mischungen umfassend mindestens zwei der genannten Gelbildner in der jeweiligen Zusammensetzung vorliegen. Bevorzugt wird Gelatine eingesetzt, die erfindungsgemäss mit einem Weichmacher wie Glycerin oder einem anderen Polyol versetzt ist. Die Zugabe des Weichmachers verbessert die Handlingseigenschaften der Gelatine. Erfindungsgemässe Zusammensetzungen umfassen vorzugsweise als Gelbildner Gelatine und ein Polyol, wie Glycerin und/oder deren Umsetzungsprodukte, optional in Gegenwart von Wasser. Alternativ können auch Gelatine und ein Weichmacher, wie Sorbitol, eingesetzt werden. Der Weichmacher bewirkt eine Erhöhung des Schmelzbereiches durch Ausbildung von intermolekularen Wasserstoffbrückenbindungen.

Als Gelbildner wird erfindungsgemäss Gelatine vorzugsweise (denaturiertes Kollagen, tierisches Eiweiss, Protein), besonders bevorzugt wird ein sauer hydrolysiertes Kollagen, oder Gelatine und ein Polyol, wie Glycerin, eingesetzt. Alternativ können auch Casein, Stärkemehl, Cellulose, HPMC, Gummi Arabicum, Galactomannane, Guarkernmehl, Konjak, Xanthan, Calciumalginat, Dextran, Scleroglucan, Pektin, Carragenan (K-, l- und λ-Carrageen), Agar-Agar, Alginat, Alginsäure, Natriumalginat, Calciumalginat, Traganth als Gelbildner eingesetzt werden, wobei Gelatine oder Mischungen mit Gelatine bevorzugt sind.

Die Carbonsäuren werden vorzugsweise ausgewählt aus Fruchtsäuren, wie α-Hydroxycarbonsäuren, wie Äpfelsäure, Zitronensäure, Glycolsäure, Milchsäure und Weinsäure; Aminosäuren, Fettsäuren, Hydroxycarbonsäuren, Dicarbonsäuren und Mischungen umfassend mindestens zwei der genannten Säuren und/oder das Puffersystem umfasst Carboxylate von Alkylcarbonsäuren, Fettsäuren, Fruchtsäuren, Fumarate, Aminosäuren, Hydroxycarbonsäuren, Dicarbonsäuren und Mischungen umfassend mindestens zwei der genannten Säuren oder Phosphatpuffer. Für die Puffersysteme werden vorteilhaft Alkali- und/oder Erdalkali-Salze oder Zink-Salze eingesetzt.

Die Puffersysteme umfassen EDTA, TRIS: Tris(hydroxymethyl)-aminomethan für pH 7,2 bis 9,0, HEPES: 4-(2-Hydroxyethyl)-1-piperazinethanesulfonsäure für pH 6,8 bis 8,2, HEPPS: 4-(2-Hydroxyethyl)-piperazin-1-propansulfonsäure für pH 7,3 bis 8,7, Barbital-Acetat Puffer, MES: 2-(N-Morpholino)ethansulfonsäure für pH 5,2 bis 6,7, Kohlensäure-Bicarbonat-System für pH 6,2 bis 8,6; neutral, Kohlensäure-Silicat-Puffer für pH 5,0 bis 6,2; schwach sauer, Essigsäure-Acetat-Puffer für pH 3,7 bis 5,7, Phosphatpuffer: NaH₂PO₄ + Na₂HP0₄ für pH 5,4 bis 8,0, Ammoniakpuffer NH₃ + H₂O + NH₄Cl für pH 8,2 bis 10,2, Zitronensäure- oder Zitratpuffer. Besonders bevorzugte Puffersysteme umfassend Milchsäure Puffersysteme, EDTA, oder Barbital-Acetat Puffer und TRIS (Tris(hydroxymethyl)-aminomethan) Puffer.

Erfindungsgemäss einsetzbare Phosphate zur Herstellung der Phosphat enthaltenden Mineralisationsmatrices umfassen die Phosphate, Hydrogenphosphate, oder zu wasserlöslichen Phosphat-Ionen hydrolysierbare Phosphate umfassend a) Alkaliphosphate, Erdalkaliphosphate, Dihydrogenphosphate, Natriumdihydrogenphosphat, NaH₂PO₄, Kaliumdihydrogenphosphat, KH₂PO₄ Hydrogenphosphate, Dikaliumhydrogenphosphat, K₂HPO, Dinatriumhydrogenphosphat, Na₂HPO₄, Phosphatester, Monoester, Diester und Triester von Phosphaten, Natriumphosphat, Na₃PO₄, Kaliumphosphat, K₃PO₄, Calciumdihydrogenphosphat, Ca(H₂PO₄)₂, Monoester, Diester und Triester Calciumhydrogenphosphat, CaHPO₄, Calciumphosphat, Ca₃(PO₄)₂ und/oder b) die Calcium-Ionen oder Calcium-Ionen freisetzende Verbindungen umfasst Calciumchlorid, Calciumdichlorid-Dihydrat, Calcium-Salz einer Carbonsäure umfassend Alkylcarbonsäuren, Hydroxycarbonsäure, Dicarbonsäuren, Fruchtsäuren, Aminosäuren, wie Calciumlactat, Calciumgluconat, Calciumlacto-Gluconat, Calciumalginat, Calcium-L-Ascorbat, in Wasser schlecht lösliche Calcium-Ionen retardiert freisetzende Verbindungen umfassend Calciumsulfat, Calciumapatit, Calciumcarbonat, Calciumoxalat, Calciumphosphat, Calciumalginat, vorzugsweise mit einer Partikelgrösse von kleiner 100 µm, bevorzugt um 10 µm,besonders bevorzugt kleiner gleich 5 µm bspw. bis 1 µm oder 50 nm oder vorzugsweise Gemische von wasserlöslichen und schlecht in Wasser löslichen Calcium-Ionen oder Calcium-Ionen freisetzenden Verbindungen. Die in Wasser schlecht löslichen Calcium-Ionen retardiert freisetzenden Verbindungen werden zur Texturverbesserung der teilweise klebrigen Gele der gut wasserlösliche Calcium-Ionen enthaltenden Zusammensetzung zugesetzt. In Bezug auf die Gesamtzusammensetzung der Zusammensetzung können 1 bis 50 Gew.-% in Wasser schlecht lösliche Calciumfreisetzende Verbindungen eingesetzt werden, bevorzugt werden 5 bis 30 Gew.-% eingesetzt.

Die Phosphat-Ionen enthaltende Zusammensetzung enthält unter anderem ein wasserlösliches Phosphat-Salz. Geeignet sind z.B. Alkalisalze wie Natrium- oder Kaliumphosphate, Hydrogen- oder Dihydrogenposphate. Die Aufzählung ist einschliesslich aber nicht ausschliesslich. Die Konzentration der Phosphatsalze in der Zusammensetzung beträgt von 0,05 mol/l bis 4 mol/l Zusammensetzung, insbesondere Gel, bevorzugt 0,5 mol/l bis 1,5 mol/l, besonders bevorzugt um 1 mol/l plus/minus 0,5 mol/l. Die Phosphat-Ionen enthaltende Zusammensetzung enthält ausserdem ein wasserlösliches Fluoridsalz z.B. ein Alkalisalz, oder Zinnfluorid oder Olafluor. Die Aufzählung ist einschliesslich aber nicht ausschliesslich. Die Konzentration des Fluorids liegt in der Zusammensetzung von 0 bis 6000 Gew.-ppm, bevorzugt 200 bis 4000 Gew.-ppm, besonders bevorzugt 2500 bis 4000 Gew.-ppm oder um 3000 Gew.-ppm plus/minus 500 Gew.-ppm. Der pH-Wert der Phosphat-Zusammensetzung liegt vorzugsweise von 2,0 bis 8,0, bevorzugt von 3,5 von 5,5 und wird mit einem geeigneten Puffersystem eingestellt. Besonders geeignet sind Carbonsäuren wie Ascorbinsäure, Brenztraubensäure, Weinsäure, Essigsäure, Milchsäure oder Äpfelsäure aber auch alle anderen Puffersysteme. Die Konzentration des Puffers liegt von 0,25 mol/l bis 4,0 mol/l, bevorzugt von 0,5 mol/l bis 1.5 mol/l.

Zur Herstellung der Gelbildner enthaltenden Zusammensetzungen können der jeweiligen Zusammensetzung Gelatine und Glycerin zugesetzt werden. Die Menge an Gelatine beträgt vorzugsweise 25 bis 40 Gew.-% und die Menge an Glycerin 5 bis 20 Gew.-% in Bezug auf die Gesamtzusammensetzung in einer Zusammensetzung, insbesondere einer Wasser enthaltenden Zusammensetzung. Um die Komponenten homogen zu mischen, wird die Zusammensetzung auf 40 bis 90°C erwärmt, bevorzugt auf 50 bis 70°C. Die Schichtdicke der Phosphat-Ionen-Zusammensetzung in dem Verfahren oder dem der Zusammensetzung auf dem Zwischenprodukt beträgt hierbei von 50 µm bis 3000 µm,bevorzugt von 200 µm bis 2000 µm,besonders bevorzugt 300 µm bis 1500 µm.

In bevorzugten Alternativen umfassen die Zusammensetzungen jeweils unabhängig 5 bis 50 Gew.-% Gelatine in Bezug auf die Gesamtzusammensetzung und 0 bis 30 Gew.-% Glycerin in Bezug auf die Gesamtzusammensetzung, bevorzugt sind 25 bis 40 Gew.-% Gelatine und 5 bis 20 Gew.-% Glycerin in der Phosphat-Ionen enthaltenden Zusammensetzung, und 20 bis 40 Gew.-% Gelatine und 15 bis 25 Gew.-% Glycerin in der Calcium-Ionen enthaltenden Zusammensetzung. Die Gelatine enthaltenden Zusammensetzungen werden vorzugsweise auf 40 bis 90 °C erwärmt, um die Komponenten homogen zu vermischen, bevorzugt ist der Temperaturbereich von 50 bis 70 °C. Nachfolgend können die Zusammensetzungen abkühlen, wobei sie sich verfestigen.

Die Calcium-Ionen enthaltende Zusammensetzung umfasst vorzugsweise ein wasserlösliches Calciumsalz, z.B. Calciumchlorid oder Calciumlactat oder Calciumgluconat oder Calciumlacto-Gluconat. Die Aufzählung ist einschliesslich aber nicht ausschliesslich. Die Konzentration liegt von 0,1 mol/l bis 2,0 mol/l, bevorzugt von 0,5 mol/l bis 1,5 mol/l. Der pH-Wert von 4,0 bis 14,0, bevorzugt von 6,0 bis 11,0 und wird mit einem geeigneten Puffersystem eingestellt. Besonders geeignet sind Carbonsäuren, wie Ascorbinsäure, Brenztraubensäure, Weinsäure, Essigsäure, Milchsäure oder Äpfelsäure aber auch alle anderen Puffersysteme mit geeignetem pks-Wert können verwendet werden.

Die Konzentration des Puffers liegt von 0,1 mol/l und 3,0 mol/l, bevorzugt von 0,25 mol/l bis 1,0 mol/l. In dieser Zusammensetzung beträgt der Gehalt an Gelatine vorzugsweise 20 bis 40 Gew-% in Bezug auf die Gesamtzusammensetzung und die Menge an Glycerin 15 bis 25 Gew-%. Da die Calcium-Gelatine-Zusammensetzung auch nach Gelieren sehr klebrig ist und damit unangenehm im Handling wird zur Texturverbesserung ein schlecht lösliches Calcium-salz zugegeben. Besonders geeignet sind Calciumsulfat, Calciumapatit, Calciumcarbonat, Calciumoxalat. Die Aufzählung ist einschliesslich aber nicht ausschliesslich.

Die Schichtdicke der Calcium-Ionen enthaltenden Zusammensetzung in dem erfindungsgemässen Verfahren oder dem Zwischenprodukt beträgt vorzugsweise von 10 µm bis 1 cm, insbesondere 10 µm bis 5 mm oder bis 3000 µm,bevorzugt 100 µm bis 3000 µm,besonders bevorzugt 500 µm bis 3000 µm,vorzugsweise 500 µm bis 1500 µm.

Zur Herstellung der Zwischenprodukte oder zur Durchführung des Verfahrens werden die noch nicht verfestigten Zusammensetzungen, insbesondere das erste, zweite und optional weitere Gele geformt und anschliessend verfestigt.

Die Phosphat-, Calcium- und Fluorid-Ionen enthaltenden Zusammensetzungen gemäss der Erfindung können hergestellt werden wie in EP1509189A1, EP1927338A1, EP1927334A1 und/oder EP1809233A1 offenbart.

Daher ist ebenfalls Gegenstand der Erfindung ein Verfahren, indem in mindestens einem Schritt jeweils eine nicht verfestigte Zusammensetzung auf einen Bereich des prothetischen Formteils aufgetragen wird, optional können weitere nicht verfestigte Zusammensetzungen auf diese Zusammensetzung aufgetragen werden.

Ebenfalls Gegenstand der Erfindung ist ein Zwischenprodukt, umfassend ein prothetisches Formteil, wobei das Formteil zumindest bereichsweise an seiner Oberfläche mindestens eine Gelschicht (erste Gelschicht) einer Phosphat-Ionen enthaltenden Zusammensetzung aufweist. Vorzugsweise weist das Zwischenprodukt optional auf dieser ersten Gelschicht eine weitere Gelschicht, und optional eine Gelschicht einer Calcium-Ionen enthaltenden Zusammensetzung, auf. Das erfindungsgemässe Zwischenprodukt kann somit eine erste Phosphat-Ionen enthaltende Gelschicht, eine zweite Gelschicht und eine dritte Calcium-Ionen enthaltende Gelschicht aufweisen. Alternativ weist es eine erste Phosphat-Ionen enthaltende Gelschicht und eine zweite Calcium-Ionen enthaltende Gelschicht oder alternativ eine Phosphat- und Calcium-Ionen enthaltende Gelschicht auf. Fluorid kann, wie vorstehend beschrieben, in mindestens einer der Zusammensetzungen enthalten sein.

Die Erfindung wird anhand der Figuren näher erläutert, ohne die Erfindung auf den Gegenstand der Figuren zu beschränken.

Die Figuren stellen schematisch dar:
Figur 1a: Einen zahnlosen Bereich 0 in den eine Krone 4 eingefügt werden soll,
Figur 1b: Den zahnlosen Bereich, in den ein Implantat 1 mit einem Verbindungselement 2 in den Knochen eingebracht wurde,
Figur 1c: Implantat 1 mit Verbindungselement 2, Aufbauelement 3 und Krone 4.
Figur 2: Querschnitt (ohne Perspektive) eines Kieferbereiches umfassend Zahnfleisch 5a, Gingiva 5a und Kieferknochen 5b, der den Bereich 6 des Verbindungselementes 2 oder Implantats 1 (jeweils prothetisches Formteil) im Zahnfleisch 5a der epithelialen Manschette (Saumepithel) zeigt.
Figur 3: Typischer Aufbau einer prothetischen Zahnversorgung 8 umfassend ein Implantat 1, ein Verbindungselement 2, den oberen, äusseren prothetischer Aufbau 4, z.B.
Zahnkrone, Suprastruktur optional mit äusserer Beschichtung, den Bereich 6, insbesondere (Saumepithel) des Implantats und/oder Verbindungselementes im Zahnfleisch sowie eine Fixierungsschraube 7.
Figur 4: Stellt verschiedene Einbausituationen von Implantaten 1 im Kieferknochen mit und ohne Verbindungselement 2 dar.
Figur 5: Zeigt eine Vielzahl an prothetischen Versorgungen mit Kronen 4 umfassend erfindungsgemässe Formteile, wie Verbindungselement 2 (Abutment, Spacer, Pfeiler, Pfosten, Implantatschulter etc.) ggf. mit Verbindungsschraube.
Figur 6: Stellt verschiedene Einbausituationen von Implantaten 1 im Kieferknochen mit und ohne Verbindungselement 2 dar, wobei die mit A gekennzeichneten Oberflächen der Verbindungselemente mikromechanische Verankerungsstellen mit einer biomimetisch abgeschiedenen Apatitschicht aufweisen.
Figur 7: Querschnitt (ohne Perspektive) eines Kieferbereiches umfassend Zahnfleisch 5a, Gingiva 5a und Kieferknochen 5b, der den Bereich 6 des Verbindungselementes 2 oder Implantats 1 (jeweils prothetisches Formteil) im Zahnfleisch 5a der epithelialen Manschette (Saumepithel) zeigt, wobei die mit A gekennzeichneten Oberflächen der Verbindungselemente mikromechanische Verankerungsstellen mit einer biomimetisch abgeschiedenen Apatitschicht aufweisen.
Figuren 8a bis 8e: Titanoberfläche; Figur 8a: Nicht vergrössert und Figuren 8b bis 8e: Vergrössert (Balken = 1000 Mikrometer (Fig. 8b), = 50 Mikrometer (µm) (Fig. 8c), 30 Mikrometer (Fig. 8d) und 20 Mikrometer (Fig. 8e)).
Figur 9a: Halbseitige 7-fache Beschichtung, Schichtdicke 14 bis 30 µm
Figur 9b: Schichtdicke der biomimetischen Apatitschichten: X1: ca. 14 µm, X2: ca. 30 µm
Figur 10: REM-Aufnahme der biomimetischen Apatitschichten (weitgehend parallele Anordnung der fast senkrechten Nadeln; Balken = 50 µm)
Figur 11: Vergrösserung der abgeschiedenen biomimetischen Apatitschichten

### Ausführungsbeispiele

Im Folgenden ist die Herstellung der Gele im durchgeführten Experiment beschrieben. Grundsätzlich ist die Vernetzung mit GDA (Glutardialdehyd) für *in vitro*-Beschichtung nicht notwendig, aber optional möglich.

2k-Verfahren Beispiel: (Rezeptur für die Beschichtung von Ti-Plättchen mit mikromechanischen Verankerungsstellen.

### Vorbehandlungszusammensetzung:

Für die Vorbehandlungszusammensetzung wird eine 1 molare Calciumchloridlösung mit 0,1 mol Tris-Puffer versetzt und auf den pH-Wert von 9,0 eingestellt.

### Ca-Ionen enthaltende Zusammensetzung:

(i) Für das Ca-Gel: Lösen von 147 g CaC1₂×2H₂O mit 47,5 g Milchsäure in 800 ml Wasser. Mit 106 ml 5 N NaOH wird ein pH-Wert von 10.5 eingestellt. Zur Herstellung des Gels werden 18 ml dieser Lösung mit 6 g Glycerin und 8 g Calciumsulfat und 13,6 g 300 Bloom-Gelatine vermischt und erwärmt. Das flüssige Gel wird mit einer Rakel auf eine Dicke von 1 mm ausgestrichen oder in einer Schablone mit der Wandstärke von 1 mm gepresst. Nach Verfestigung werden die Streifen in 1x1 cm grosse Quadrate geschnitten.
(ii) 2 g einer 25%igen GDA-Lösung (Glutardialdehyd) werden mit Wasser auf 100 ml aufgefüllt und die Gelquadrate für 20 s darin gebadet. Anschliessend wird die anhaftende Flüssigkeit vorsichtig verblasen. Die Gele werden nun von der anderen Seite analog behandelt. Die Gelquadrate werden in Alubeutel eingeschweisst und direkt vor der Anwendung einzeln entnommen.

### Phosphat-Ionen enthaltende Zusammensetzung:

(i) 59 g Na₂HPO₄ werden mit 91 g Milchsäure, 6,6 g Olaflur, 6 ml 5 N NaOH, 300 ml Wasser auf einen pH-Wert von 4,0 eingestellt und auf 500 ml aufgefüllt. 24 ml der Lösung werden mit 6 g Glycerin und 10 g einer 300 Bloom Schweineschwarte-Gelatine unter Erwärmen zu einer dickflüssigen Lösung verarbeitet. In eine Schablone mit der Wandstärke von 500 µm wird etwas Flüssigkeit eingebracht und unter 2 bar Druck gepresst. Nach Verfestigung werden die Streifen der Schablone entnommen und in 1x1 cm grosse Quadrate geschnitten.
(ii) 1.5 g einer 25%igen GDA-Lösung werden mit Wasser auf 100 ml aufgefüllt und die Gelquadrate für 20 s darin gebadet. Anschliessend wird die anhaftende Flüssigkeit vorsichtig verblasen. Die Gele werden nun von der anderen Seite analog behandelt. Die Gelquadrate werden in Alubeutel eingeschweisst und direkt vor der Anwendung einzeln entnommen.

### Beschichtung von Ti-Plättchen:

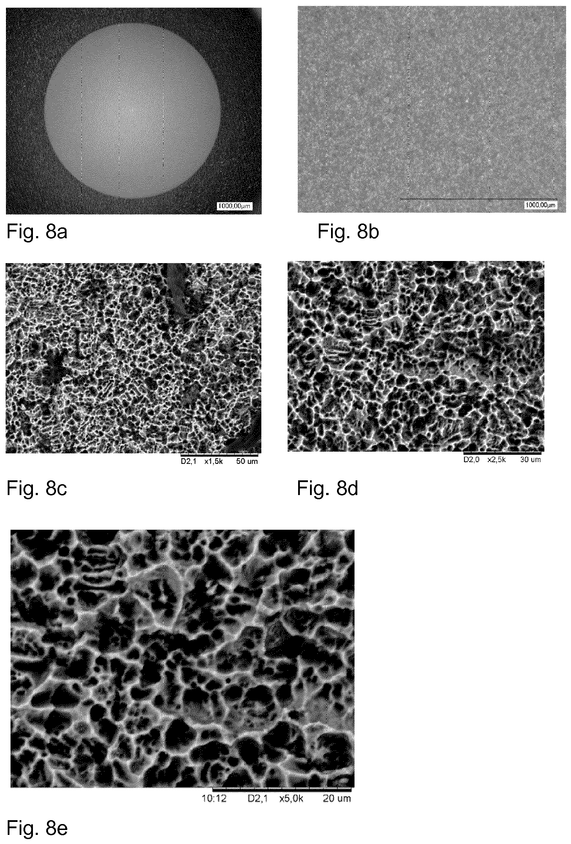

Die Oberfläche der Ti-Plättchen wurde zuvor im nicht-thermischen Plasma (kaltes Plasma) behandelt. Die Figuren 8a bis 8e zeigen die Titanoberfläche, Figur 8a nicht vergrössert und die Figuren 8b bis 8e zeigen die stark poröse Oberfläche vergrössert (Balken = 1000 µm (Fig. 8b), = 50 µm (Fig. 8c), = 30 µm (Fig. 8d) und = 20 µm (Fig. 8e)).Die Oberfläche weist flächendeckend verteilt, mikromechanische Verankerungsstellen in Form einer porösen Oberfläche auf. Die Porendurchmesser liegen in einem Bereich von etwa 0,5 µm bis 20 µm.

Zur Bewertung der Mineralisationsaktivität werden je 6 Titan-Plättchen mit der Vorbehandlungszusammensetzung behandelt und mit je einem Stück Phosphat-Gel und einem Stück Calcium-Gel bedeckt. Um die morphologische Änderung der Tintanoberfläche deutlich zu machen, wurde eine Scheibe zuvor zur Hälfte abgeklebt, so dass sie nur hälftig remineralisieren kann. Die Proben werden im Klimaschrank bei 37°C und 95 % Luftfeuchtigkeit aufbewahrt und nach 8 bis 12 Stunden mit lauwarmen Wasser und einer weichen Zahnbürste gereinigt.

Nach 7, 10, 12 und 17 Tauschzyklen wurden mittels 3-D-Mikroskop (Keyence) Bilder der Oberfläche aufgenommen und die Schichtdicke aus der Höhendifferenz zwischen beschichteter und unbeschichteter Seite bestimmt. Anschliessend wurde die Probe mit 4000er Schleifpapier vorsichtig hochglanzpoliert und erneut die Schichtdicke bestimmt. Die Vollständigkeit der Beschichtung wurde mittels REM untersucht.

| **Anzahl Tauschzyklen** | **Schichdicke biomimetischer Apatit** [µm] | |
|---|---|---|
| 1 | Nahezu geschlossene Beschichtung | |
| 7 | 14 bis 31 | |
| 10 | 18 bis 42 | |
| 12 | 18 bis 50 | |
| 17 | 20 bis 60 | |
| 21* | 24 bis 30 | mittel 28 [µm] |

| | | |
|---|---|---|
| * nach 17 Tauschzyklen erfolgt Polieren anschliessend weitere 4 Tauschzyklen | | |

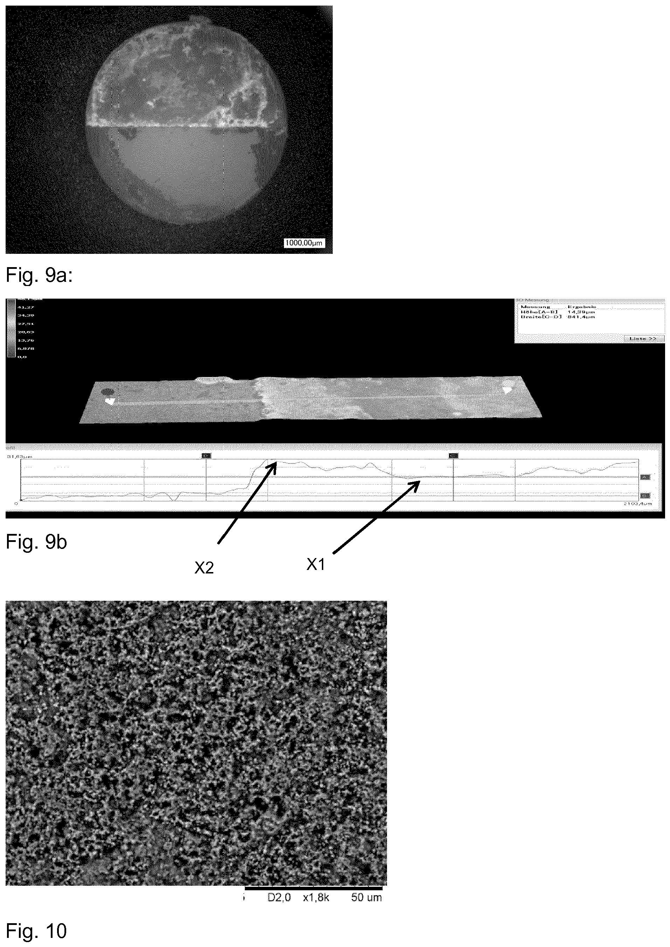

In den Figuren 8a bis 8e ist die noch unbeschichtete Titanoberfläche mit mikromechanischen Verankerungsstellen in Form einer stark porösen Oberflächenstruktur umfassend Porendurchmesser von 1 bis 7 µm, siehe Fig. 8e, in unterschiedlichen Vergrösserungen dargestellt. Die Figuren 9a und 9b zeigen halbseitig mit biomimetischem Apatit beschichtete Titanoberflächen nach 7 Tauschzyklen. Die Schichtdicke der Apatitschichten liegt bei 14 bis 30 µm. Figur 10 zeigt eine REM-Aufnahme der erfindungsgemässen biomimetisch abgeschiedenen Apatitschichten und die weitgehend parallele Anordnung der fast senkrechten Nadeln. Die Anordnung der Kristallitnadeln zu Apatitschichten des biomimetischen Apatits ist in der REM-Aufnahme der Figur 11 gut zu erkennen.

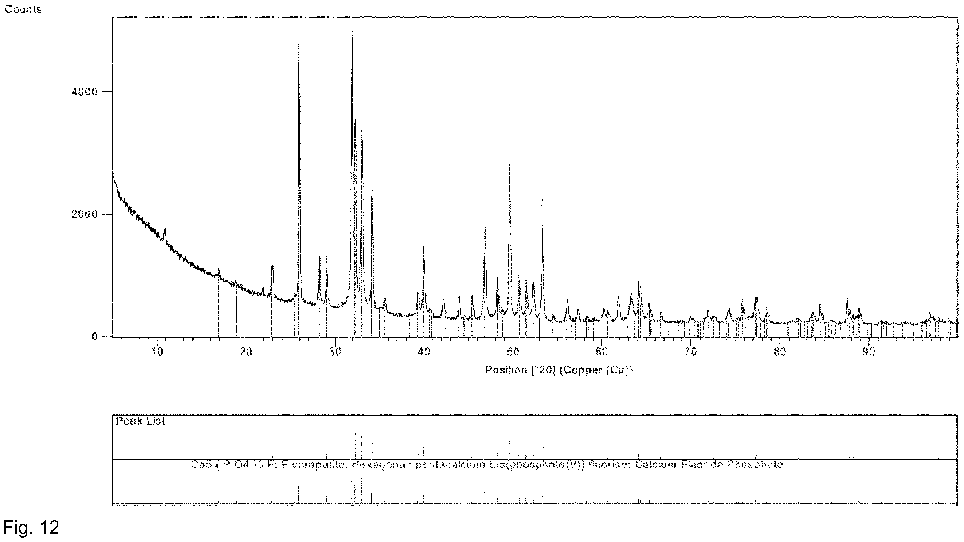

Von den biomimetisch abgeschiedenen Fluor-Apatitschichten wurden XRD-Diffraktogramme (5° bis 100° (2Theta) in Reflexion mit einem X'Pert Pro MPD aufgenommen, die eindeutig kristallinem Fluorapatit zugeordnet werden können. Figur 12 zeigt ein XRD-Diffraktogramm von Fluorapatit aus Gelatine auskristallisiert als Fluorapatit mit Gelatine (Strahlungsquelle: Kupfer (Cu). Die XRD-Linien können eindeutig einem Ca₅(PO₄)₃F, Fluorapatit (hexagonal) zugeordnet werden. Anschließend wurden die Proben im Elementaranalysator LECO "RC-612" bezüglich des durch die im biomimetisch abgeschiedenen Fluorapatit vorliegende Gelatine untersucht. Der Kohlenstoffgehalt im biomimetisch abgeschiedenen Fluorapatit konnte auf circa 0,5 Gew.-% bestimmt werden.

### Bezugszeichenliste:

0 Zahnloser Bereich im Kiefer
1 Implantat
2 Verbindungselement (Abutment, Spacer, Pfeiler, Pfosten, Implantatschulter etc.) ggf. mit Verbindungsschraube
3 Aufbauelement
4 oberer, äussere prothetischer Aufbau, z.B. Zahnkrone, Suprastruktur optional mit äusserer Beschichtung
5 Kieferbereich umfassend Zahnfleisch und Kieferknochen;
**5a** Zahnfleisch, Gingiva; **5b**: Knochen
6 Bereich des Implantats und/oder Verbindungselementes im Zahnfleisch
7 Fixierungsschraube
8 prothetische Versorgung umfassend 1, 2 optional 3, sowie 4, 6 und 7
A Oberfläche mit mikromechanischen Verankerungsstellen, wie beispielsweise einem porösen Bereich, einem aufgerauten, geätzten oder mechanisch mit Feststoffpartikeln behandelten Bereich, und mit mindestens einer biomimetisch abgeschiedenen Apatitschicht.

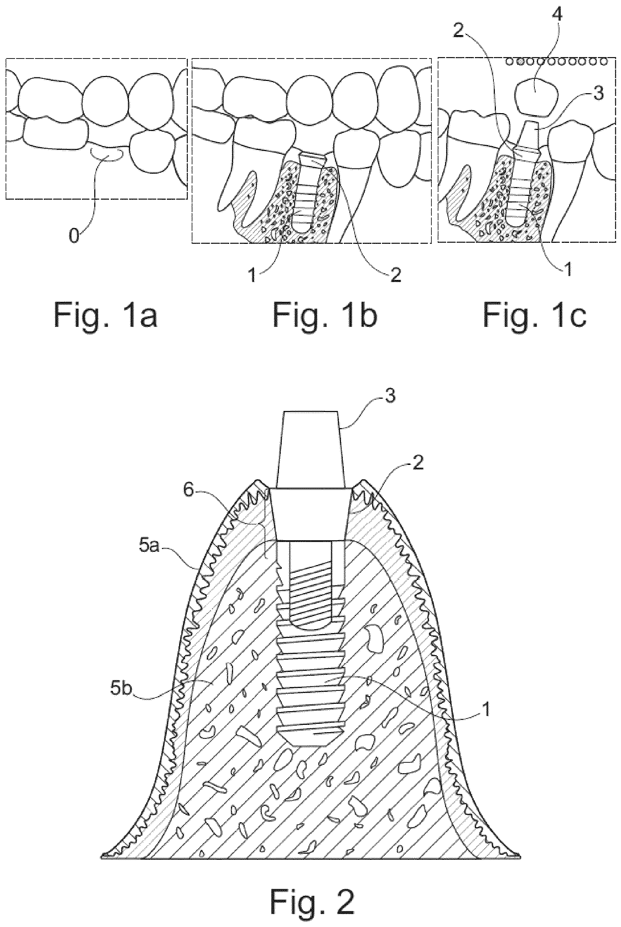

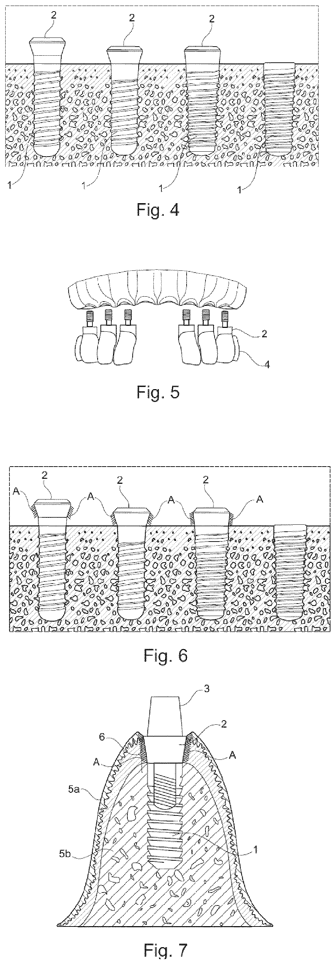

Die erfindungsgemässen prothetischen Formteile umfassend eine Apatitschicht, die vorzugsweise im Patienten in der epithelialen Manschette (Saumepithel) angeordnet ist, dient dazu einen zahnlosen Bereich **0,** wie in Figur 1a dargestellt mit einer Implantat **1** getragenen Krone **4** (Figur 1b und 1c) dauerhaft und langlebig biologisch zu verbinden. Diese Verbindung ist dynamisch. Sie kann daher aufgebrochen werden und kann erneut zusammenwachsen. Mit den erfindungsgemässen prothetischen Formteilen ist es möglich die Formteile an Gingiva-Epithelzellen in der epithelialen Manschette (Saumepithel) mittels Hemidesmosomen anzuheften. Durch diese Massnahme kann eine bakterielle Kontamination im Bereich des Implantates vermindert werden und eine biologische Verbindung an dieser Kontaktstelle erhalten werden. Die Figur 2 zeigt eine Situation ohne Apatitschicht und mit erfindungsgemässer Apatitschicht in Figur 7. Im Bereich des Zahnfleisches **5a** der epithelialen Manschette (Saumepithel) **6** weist das erfindungsgemässe prothetische Formteil, hier das Verbindungselement **2**, eine erfindungsgemässe biomimetisch abgeschiedene Apatitschicht auf. Diese biomimetisch abgeschiedene Apatischicht enthält vorzugsweise einen Gehalt an Aminosäuren, Aminosäurederivate, Proteine, denaturiertem Kollagen, insbesondere umfasst das Apatit Bestandteile von Kollagen, denaturiertem Kollagen, Gelatine, Proteinketten, Gelatine-Glycerin-Gel aus den Zusammensetzungen aus denen das Apatit abgeschieden wird.

Die Figuren 4 und 6 stellen verschiedene Einbausituationen von Implantaten 1 mit unterschiedlichen Verbindungselementen 2 dar, wobei in Figur 6, die Verbindungselemente 2 unterschiedliche mit A gekennzeichnete Bereiche der Oberflächen aufweisen. Diese Bereiche A weisen mikromechanische Verankerungsstellen auf, wie beispielsweise einen porösen Bereich, einen aufgerauten, geätzten oder mechanisch mit Feststoffpartikeln behandelten Bereich. An diesen mikromechanischen Verankerungsstellen ist eine biomimetische Apatitschicht von mindestens 1 µm (Mikrometer) bis 100 µm oder bis 1 mm aufgewachsen. Erfindungsgemäss wird die biomimetische Apatischicht durch Kristallisation von Apatit aus den vorgenannten Zusammensetzungen, insbesondere Gelen, erhalten. Der biomimetische Apatit wird später im Mund des Patienten durch eine zelluläre Anheftung, insbesondere über Hemidesmosome, mit den Epithelzellen der Gingiva, insbesondere der epithelialen Manschette (Saumepithel) verbunden.

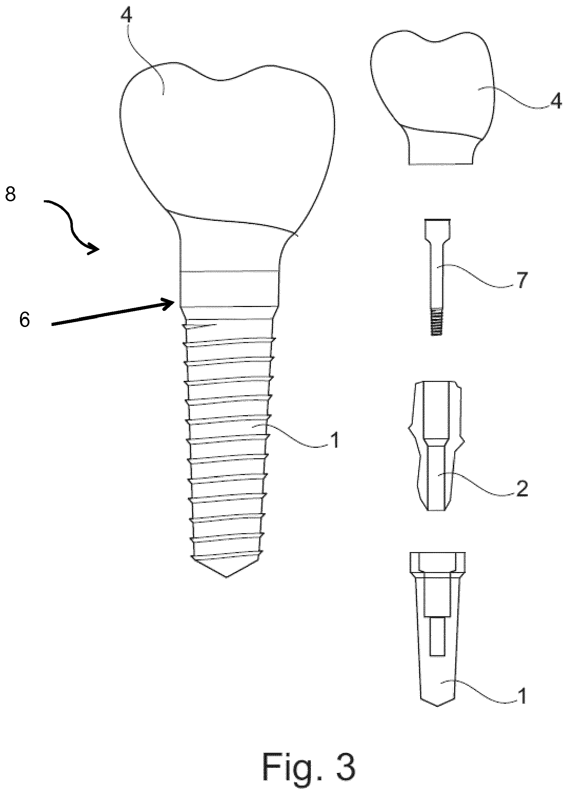

Die Figuren 3 und 5 stellen typische Formteile einer prothetischen Zahnversorgung dar, wobei die Figur 3 die einzelnen Formteile und Figur 5 die zusammengebauten prothetischen Formteile zum Einbau in einen Oberkiefer darstellt.

Dem Fachmann ist klar, dass erfindungsgemäss als prothetische Formteile alle geeigneten Formteile aus jeglichen physiologisch geeigneten Materialien mit einer biomimetischen Apatitschicht versehen werden können. Als prothetische Formteile können ebenfalls resorbierbare Formteile eingesetzt werden.

## Patentansprüche

1. Prothetisches Formteil, wobei
das Formteil zumindest bereichsweise an seiner Oberfläche mindestens eine Schicht biomimetischen, kristallinen Apatit ausgewählt aus Fluorapatit, Hydroxylapatit oder deren Gemischen aufweist, wobei die Oberfläche mindestens in diesem Bereich mikromechanische Verankerungsstellen aufweist und
wobei zwischen den Schichten des Apatits oder in Kavitäten organische Verbindungen des Gelbildners, vorzugsweise der Gelatine, eingelagert sind, **dadurch gekennzeichnet, dass** der Apatit mindestens einen Kohlenstoffgehalt im Bereich von 0,25 bis 2,5 Gew.-% und einen Stickstoffgehalt im Bereich von 0,09 bis 0,9 Gew.-% aufweist.

2. Formteil, nach Anspruch 1, **dadurch gekennzeichnet, dass** die Schichtdicke der mindestens einen Apatitschicht mindestens 1 µm beträgt.

3. Formteil nach Anspruch 1 oder 2, **dadurch gekennzeichnet, dass** das Formteil ein enossales Implantat (1), dentales, enossales (intraossär) Implantat (1), Verbindungselement (2), Aufbauelement (3), dentale Hülse, Abutment (2), Superstruktur (4), Teil einer dentalen Prothese, dentale Totalprothese, orthopädische Prothese oder Teile davon, künstlicher Zahn, Veneer, Inlay, Onlay, dentale Trägerstruktur (2,3), Brücke (4), Krone (4), Unterfütterung, Prothesensattel, Knochenprothese, Gelenkprothese, Revisions-Total-Gelenkendoprothese und/oder Spacer ist.

4. Formteil nach einem der Ansprüche 1 bis 3, **dadurch gekennzeichnet, dass** das Formteil ein Verbindungselement (2), ein Aufbauelemente (3), dentales, enossales Zahnimplantat (1) und/oder ein Implantatpfosten (2) ist.

5. Formteil nach einem der Ansprüche 1 bis 4, **dadurch gekennzeichnet, dass** das Formteil aus Titan, Titanlegierung, Titanoxid, Kobalt-Chrom-Legierung, CoCrMo-Legierung, Gold, dentaler Keramik, Zirkonoxid, Lithiumdisilikat, Polymer, Polymergemisch, dentalem Prothesenkunststoff gebildet ist.

6. Formteil nach einem der Ansprüche 1 bis 5, **dadurch gekennzeichnet, dass** der biomimetische Apatit umfasst Aminosäuren, Aminosäurederivate, Proteine, denaturiertes Kollagen.

7. Formteil nach einem der Ansprüche 1 bis 6, **dadurch gekennzeichnet, dass** mindestens ein Bereich der Oberfläche des Formteils mikromechanische Verankerungsstellen aufweist, die umfassen eine poröse und/oder raue Oberflächentopographie.

8. Formteil, nach einem der Ansprüche 1 bis 7, **dadurch gekennzeichnet, dass**
a) die Oberfläche des prothetischen Formteils bereichsweise in dem Bereich, der später im Bereich des Zahnfleisches (6) angeordnet ist, mikromechanische Verankerungsstellen aufweist,
b) die Oberfläche des prothetischen Formteils bereichsweise in dem Bereich, der später im Bereich von Epithelzellen, dem Zahnfleisch, der Gingiva, Gingiva-Epithelzellen, Fibroblasten oder dem Bereich der epithelialen Manschette (Saumepithel) angeordnet ist, mikromechanische Verankerungsstellen aufweist, wobei das prothetische Formteil ausgewählt ist aus Verbindungselement (2), Aufbauelement (3), Abutment (2), Implantat (1), oberer, äusserer prothetischer Aufbau (4), Krone (4), Superstruktur (4), enossales Implantat (1), dentales enossales (intraossär) Implantat (1), dentale Hülse, Teil einer dentalen Prothese, Teil einer Knochen-Prothese, dentale Totalprothese, orthopädische Prothese oder Teile davon, künstlicher Zahn, Veneer, Inlay, Onlay, dentale Trägerstruktur (2,3), Brücke (4), Unterfütterung, Prothesensattel, Knochenprothese, Gelenkprothese, Revisions-Total-Gelenkendoprothese und/oder Spacer.

9. Verfahren zur Abscheidung von biomimetischem, kristallinem Apatit ausgewählt aus Fluorapatit, Hydroxylapatit oder deren Gemischen auf einem prothetischen Formteil, umfassend die Schritte
(i.a) Bereitstellen eines prothetischen Formteils, wobei mindestens ein Bereich der Oberfläche des Formteils mikromechanische Verankerungsstellen aufweist, oder
(i.b) Behandeln zumindest eines Bereiches der Oberfläche eines prothetischen Formteils, und Erhalten von mikromechanischen Verankerungsstellen, und optional (ii) Behandeln zumindest eines Bereiches der Oberfläche eines prothetischen Formteils, mit einer Vorbehandlungszusammensetzung mit einem definierten pH-Wert,
(iii) In-Kontaktbringen mindestens dieses Bereiches des prothetischen Formteils umfassend mikromechanische Verankerungsstellen mit einer Phosphat-Ionen enthaltenden Zusammensetzung, welche einen Gelbildner umfasst, wobei eine Gelschicht ausgebildet wird,
(iv) optional Auftragen einer weiteren Schicht, In-Kontaktbringen der ersten Gelschicht mit einer weiteren Zusammensetzung, die einen Gelbildner umfasst, unter Ausbildung einer weiteren Gelschicht,
(v) In-Kontaktbringen der ersten Gelschicht oder der weiteren Schicht mit einer Calcium-Ionen enthaltenden Zusammensetzung, wobei eine Gelschicht ausgebildet wird,
(vi) Abscheidung von biomimetischem, kristallinem Apatit ausgewählt aus Fluorapatit, Hydroxylapatit oder deren Gemischen, auf der Oberfläche des prothetischen Formteils, wobei zwischen den Schichten des Apatits oder in Kavitäten organische Verbindungen des Gelbildners, vorzugsweise der Gelatine, eingelagert sind und
wobei der Apatit mindestens einen Kohlenstoffgehalt im Bereich von 0,25 bis 2,5 Gew.-% und einen Stickstoffgehalt im Bereich von 0,09 bis 0,9 Gew.-% aufweist.

10. Verfahren nach Anspruch 9, **dadurch gekennzeichnet, dass**
(a) die Phosphat-Ionen enthaltende Zusammensetzung umfasst
(a.1) mindestens einen Gelbildner,
(a.2) wasserlösliche Phosphate oder zu wasserlöslichen Phosphat-Ionen hydrolysierbare Phosphate,
(a.3) optional Fluorid,
(a.4) optional eine Carbonsäure oder ein Puffersystem von pH 4 bis 7,
(a.5) optional Glycerin, und
(b) wobei die Calcium-Ionen enthaltende Zusammensetzung umfasst
(b.1) mindestens einen Gelbildner,
(b.2) Calcium-Ionen,
(b.3) optional Glycerin.

11. Verfahren nach Anspruch 9 oder 10, **dadurch gekennzeichnet, dass** das Behandeln der Oberfläche des Formteils eine mechanische, chemische, elektrochemische Behandlung und/oder ein Behandeln in einem Plasmaverfahren oder durch eine Kombination der Verfahren umfasst und ein Bereich der Oberfläche des prothetischen Formteils mit mikromechanischen Verankerunqsstellen erhalten wird und optional die Oberfläche in einem chemischen, elektrochemischen und/oder in einem Plasmaverfahren aktiviert wird.

12. Verfahren nach einem der Ansprüche 9 bis 11, **dadurch gekennzeichnet, dass** jeweils unabhängig die Phosphat-Ionen und/oder Calcium-Ionen enthaltende Zusammensetzung Aminosäuren, Derivate von Aminosäuren, Proteine, Kollagen oder denaturiertes Kollagen umfasst.

13. Verfahren nach einem der Ansprüche 9 bis 12, **dadurch gekennzeichnet, dass** der Gelbildner Gelatine umfasst.

14. Verfahren nach einem der Ansprüche 9 bis 13, **dadurch gekennzeichnet, dass** jeweils unabhängig die Phosphat-Ionen und/oder Calcium-Ionen enthaltende Zusammensetzung einen Gehalt an Wasser aufweist.

15. Zwischenprodukt, umfassend ein prothetisches Formteil, wobei mindestens ein Bereich der Oberfläche des Formteils mikromechanische Verankerungsstellen aufweist, und wobei das Formteil zumindest bereichsweise an seiner Oberfläche mindestens eine Gelschicht einer Phosphat-Ionen enthaltenden Zusammensetzung, und optional darauf eine weitere Gelschicht, und eine Gelschicht einer Calcium-Ionen enthaltenden Zusammensetzung aufweist,
- zur Abscheidung von biomimetischem, kristallinem Apatit ausgewählt aus Fluorapatit, Hydroxylapatit oder deren Gemischen, auf der Oberfläche des prothetischen Formteils, wobei zwischen den Schichten des Apatits oder in Kavitäten organische Verbindungen des Gelbildners, vorzugsweise der Gelatine, eingelagert sind und wobei der Apatit mindestens einen Kohlenstoffgehalt im Bereich von 0,25 bis 2,5 Gew. % und einen Stickstoffgehalt im Bereich von 0,09 bis 0,9 Gew.-% aufweist, zur Herstellung eines prothetischen Formteils nach einem der Ansprüche 1 bis 8.

16. Verwendung von biomimetischem, kristallinem Apatit zur mindestens bereichsweisen Beschichtung prothetischer Formteile und zur Herstellung eines prothetischen Formteils nach einem der Ansprüche 1 bis 8.

17. Verwendung einer Phosphat-Ionen enthaltenden Zusammensetzung und einer Calcium-Ionen enthaltenden Zusammensetzung nach einem der Ansprüche 9 bis 14 oder von Formulierungen enthaltend diese Zusammensetzungen zur biomimetischen Abscheidung von Apatit auf einer Oberfläche eines prothetischen Formteils nach Anspruch 1, wobei die Oberfläche mikromechanische Verankerungsstellen aufweist oder, wobei die Oberfläche des Formteils vor der Abscheidung mechanisch, chemisch, elektrochemisch und/oder mittels einer Plasmabehandlung aktiviert wurde oder zur Herstellung eines Zwischenproduktes nach Anspruch 15.

18. Verwendung nach Anspruch 17, **dadurch gekennzeichnet, dass** zumindest eine bereichsweise Abscheidung von biomimetischem Apatit erfolgt und/oder in diesem Bereich eine im Wesentlichen homogene Abscheidung von Apatit erfolgt.

19. Formteil nach einem der Ansprüche 1 bis 8 oder Zwischenprodukt nach Anspruch 15 zur Verwendung zur dentalen, prothetischen oder chirurgischen Versorgung bei Zahnverlust, insbesondere zur zellulären Anheftung von Zellen des Zahnfleisches oder Schleimhautzellen des Zahnfleisches über Hemidesmosome oder andere biologische Mechanismen an die mit dem Formteil in Kontakt tretenden Bereiche.

## Claims

1. Prosthetic moulding,
the moulding having, at least area by area, at least one layer of biomimetic crystalline apatite selected from fluoroapatite, hydroxyapatite or their mixtures on its surface, the surface having micromechanical anchoring positions at least in said area and
organic compounds of the gel forming agent, preferably of the gelatine, being embedded between the layers of the apatite or in cavities,
**characterised in that**
the apatite has at least a carbon content in the range of 0.25 to 2.5 % by weight and a nitrogen content in the range of 0.09 to 0.9 % by weight.

2. Moulding according to claim 1, **characterised in that** the layer thickness of the at least one apatite layer is at least 1 µm.

3. Moulding according to claim 1 or 2, **characterised in that** the moulding is an enossal implant (1), dental enossal (intraosseous) implant (1), connecting element (2), mounting element (3), dental sleeve, abutment (2), superstructure (4), part of a dental prosthesis, dental total prosthesis, orthopedic prosthesis or parts thereof, artificial tooth, veneer, inlay, onlay, dental supporting structure (2,3), bridge (4), crown (4), relining, denture saddle, bone prosthesis, joint prosthesis, revision total joint endoprosthesis, and/or spacer.

4. Moulding according to any one of the claims 1 to 3, **characterised in that** the moulding is a connecting element (2), a mounting element (3), dental enossal dental implant (1), and/or an implant post (2).

5. Moulding according to any one of the claims 1 to 4, **characterised in that** the moulding is formed of titanium, titanium alloy, titanium oxide, cobalt-chromium alloy, CoCrMo alloy, gold, dental ceramic, zirconium oxide, lithium disilicate, polymer, polymer mixture, dental prosthetic plastic.

6. Moulding according to any one of the claims 1 to 5, **characterised in that** the biomimetic apatite comprises amino acids, amino acid derivatives, proteins, denatured collagen.

7. Moulding according to any one of the claims 1 to 6, **characterised in that** at least one area of the surface of the moulding has micromechanical anchoring positions, which comprise a porous and/or rough surface topography.

8. Moulding, according to any one of the claims 1 to 7, **characterised in that**
a) the surface of the prosthetic moulding has micromechanical anchoring positions, area by area, in the area, which is later arranged in the area of the gums (6),
b) the surface of the prosthetic moulding has micromechanical anchoring positions, area by area, in the area, which is later arranged in the area of epithelial cells, of the gums, of the gingiva, gingival epithelial cells, fibroblasts, or the area of the epithelial cuff (junctional epithelium), the prosthetic moulding being selected from connecting element (2), mounting element (3), abutment (2), implant (1), upper outer prosthetic structure (4), crown (4), superstructure (4), enossal implant (1), dental enossal (intraosseous) implant (1), dental sleeve, part of a dental prosthesis, part of a bone prosthesis, dental total prosthesis, orthopedic prosthesis or parts thereof, artificial tooth, veneer, inlay, onlay, dental supporting structure (2,3), bridge (4), relining, denture saddle, bone prosthesis, joint prosthesis, revision total joint endoprosthesis, and/or spacer.

9. Method for depositing biomimetic crystalline apatite selected from fluoroapatite, hydroxyapatite or their mixtures on a prosthetic moulding, comprising the steps of
(i.a) providing a prosthetic moulding, at least one area of the surface of the moulding having micromechanical anchoring positions, or
(i.b) treating at least one area of the surface of a prosthetic moulding, and obtaining micromechanical anchoring positions, and optionally
(ii) treating at least one area of the surface of a prosthetic moulding, with a pretreatment composition having a defined pH value,
(iii) contacting at least said area of the prosthetic moulding comprising micromechanical anchoring positions with a composition containing phosphate ions which comprises a gel forming agent, whereby a gel layer being formed,
(iv) optionally applying a further layer, contacting the first gel layer with a further composition which comprises a gel forming agent, forming a further gel layer,
(v) contacting the first gel layer or the further layer with a composition containing calcium ions, whereby a gel layer being formed,
(vi) depositing biomimetic crystalline apatite selected from fluoroapatite, hydroxyapatite or their mixtures on the surface of the prosthetic moulding, organic compounds of the gel forming agent, preferably of the gelatine, being embedded between the layers of the apatite or in cavities, and
the apatite having at least a carbon content in the range of 0.25 to 2.5 % by weight and a nitrogen content in the range of 0.09 to 0.9 % by weight.

10. Method according to claim 9, **characterised in that**
(a) the composition containing phosphate ions comprises
(a.1) at least one gel forming agent,
(a.2) water-soluble phosphates or phosphates being hydrolysable to water-soluble phosphate ions,
(a.3) optionally fluoride,
(a.4) optionally a carboxylic acid or a buffer system of pH 4 to 7,
(a.5) optionally glycerin, and
(b) the composition containing calcium ions comprising
(b.1) at least one gel forming agent,
(b.2) calcium ions,
(b.3) optionally glycerin.

11. Method according to claims 9 or 10, **characterised in that** treating the surface of the moulding comprises mechanical, chemical, electrochemical treatment and/or treating in a plasma process or by a combination of the methods, and an area of the surface of the prosthetic moulding having micromechanical anchoring positions is obtained, and optionally the surface is activated by a chemical, electrochemical and/or in a plasma process.

12. Method according to any one of the claims 9 to 11, **characterised in that** the composition containing phosphate ions and/or calcium ions, each independently, comprises amino acids, derivatives of amino acids, proteins, collagen, or denatured collagen.

13. Method according to any one of the claims 9 to 12, **characterised in that** the gel forming agent comprises gelatine.

14. Method according to any one of the claims 9 to 13, **characterised in that** the composition containing phosphate ions and/or calcium ions, each independently, has a content of water.

15. Intermediate, comprising a prosthetic moulding, at least one area of the surface of the moulding having micromechanical anchoring position, and the moulding having, at least area by area, at least one gel layer of a composition containing phosphate ions on its surface, and optionally thereon a further gel layer, and a gel layer of a composition containing calcium ions,
- for depositing biomimetic crystalline apatite selected from fluoroapatite, hydroxyapatite or their mixtures on the surface of the prosthetic moulding, organic compounds of the gel forming agent, preferably of the gelatine, being embedded between the layers of the apatite or in cavities, and the apatite having at least a carbon content in the range of 0.25 to 2.5 % by weight and a nitrogen content in the range of 0.09 to 0.9 % by weight, for producing a prosthetic moulded part according to any one of the claims 1 to 8.

16. Use of biomimetic crystalline apatite for coating prosthetic mouldings, at least area by area, and for producing a prosthetic moulding according to any one of the claims 1 to 8.

17. Use of a composition containing phosphate ions and of a composition containing calcium ions according to any one of the claims 9 to 14, or of formulations containing these compositions, for biomimetically depositing apatite on a surface of a prosthetic moulding according to claim 1, the surface having micromechanical anchoring positions or, the surface of the moulding having been activated mechanically, chemically, electrochemically and/or by means of a plasma process prior to deposition, or for producing an intermediate according to claim 15.

18. Use according to claim 17, **characterised in that** a deposition of biomimetic apatite ensues, at least area by area, and/or an essentially homogenous deposition of apatite ensues in said area.

19. Moulding according to any one of the claims 1 to 8 or intermediate according to claim 15 for use in dental, prosthetic, or surgical treatment in case of tooth loss, in particular for cellular attachment of cells of the gums or mucosal cells of the gums via hemidesmosomes or other biological mechanisms to areas contacting the moulding.

## Revendications

1. Moulage prothétique,
le moulage ayant, au moins zone par zone, au moins une couche de l'apatite cristalline biomimétique sélectionnée parmi de la fluoroapatite, l'hydroxyapatite ou leurs mélanges sur sa surface, la surface ayant des positions d'ancrage micromécaniques au moins dans ladite zone et
des composants organiques de l'agent gélifiant, de préférence de la gélatine, étant incorporés entre les couches de l'apatite ou dans des cavités,
**caractérisé en ce que**
l'apatite a au moins une teneur en carbone dans la gamme de 0,25 à 2,5 % en poids et une teneur en azote dans la gamme de 0,09 à 0,9 % en poids.

2. Moulage selon la revendication 1, **caractérisé en ce que** l'épaisseur de couche de l'au moins une couche d'apatite est au moins 1 µm.

3. Moulage selon la revendication 1 ou 2, **caractérisé en ce que** le moulage est un implant endo-osseux (1), un implant endo-osseux (intraosseux) dentaire (1), un élément de connexion (2), un élément de montage (3), un manchon dentaire, un pilier (2), une superstructure (4), une partie d'une prothèse dentaire, une prothèse totale dentaire, une prothèse orthopédique ou des parties en eux, une dent artificielle, une facette, un inlay, un onlay, une structure de support dentaire (2,3), un bridge (4), une couronne (4), un rebasage, une selle prothétique, une prothèse osseuse, une prothèse d'articulation, une endoprothèse d'articulation totale de révision, et/ou une entretoise.

4. Moulage selon l'une des revendications 1 à 3, **caractérisé en ce que** le moulage est un élément de connexion (2), un élément de montage (3), un implant endo-osseux dentaire (1), et/ou un poteau d'implant (2).

5. Moulage selon l'une des revendications 1 à 4, **caractérisé en ce que** le moulage est formé du titane, de l'alliage de titane, de l'oxyde de titane, de l'alliage cobalt-chrome, de l'alliage CoCrMo, de l'or, de la céramique dentaire, de l'oxyde de zirconium, du disilicate de lithium, du polymère, du mélange de polymères, du plastique prothétique dentaire.

6. Moulage selon l'une des revendications 1 à 5, **caractérisé en ce que** l'apatite biomimétique comprend des aminoacides, des dérivatifs d'aminoacides, des protéines, du collagène dénaturé.

7. Moulage selon l'une des revendications 1 à 6, **caractérisé en ce qu'**au moins une zone de la surface du moulage a des positions d'ancrage micromécanique, qui comprennent une topographie de surface poreuse et/ou rugueuse.

8. Moulage selon l'une des revendications 1 à 7, **caractérisé en ce que**
a) la surface du moulage prothétique a des positions d'ancrage micromécaniques, zone par zone, dans la zone, qui est arrangée dans la zone des gencives (6) après,
b) la surface du moulage prothétique a des positions d'ancrage micromécaniques, zone par zone, dans la zone, qui est arrangée dans la zone des cellules épithéliales, des gencives, de la gencive, des cellules épithéliales gingivales, des fibroblastes, ou la zone de la coupelle épithéliale (l'épithélium jonctionnel), le moulage prothétique étant sélectionné parmi d'un élément de connexion (2), un élément de montage (3), un pilier (2), un implant (1), une structure prothétique extérieure supérieure (4), une couronne (4), une superstructure (4), un implant endo-osseux (1), un implant endo-osseux (intraosseux) dentaire (1), un manchon dentaire, une partie d'une prothèse dentaire, une partie d'une prothèse osseuse, une prothèse totale dentaire, une prothèse orthopédique ou des parties en eux, une dent artificielle, une facette, un inlay, un onlay, une structure de support dentaire (2,3), un bridge (4), un rebasage, une selle prothétique, une prothèse osseuse, une prothèse d'articulation, une endoprothèse d'articulation totale de révision, et/ou une entretoise.

9. Procédé pour déposer l'apatite cristalline biomimétique sélectionnée parmi de la fluoroapatite, l'hydroxyapatite ou leurs mélanges sur un moulage prothétique, comprenant les étapes de
(i.a) fournir un moulage prothétique, au moins une zone de la surface du moulage ayant des positions d'ancrage micromécaniques, ou
(i.b) traiter au moins une zone de la surface du moulage prothétique, et obtenir des positions d'ancrage micromécaniques, et facultativement
(ii) traiter au moins une zone de la surface d'un moulage prothétique, avec une composition de traitement préalable ayant une valeur pH définie,
(iii) contacter au moins ladite zone du moulage prothétique comprenant des positions d'ancrage micromécaniques avec une composition contenant des ions de phosphate qui comprend un agent gélifiant, selon lequel une couche de gel étant formée,
(iv) facultativement appliquer une autre couche, contacter la première couche de gel avec une autre composition qui comprend un agent gélifiant, formant une autre couche de gel,
(v) contacter la première couche de gel ou l'autre couche avec une composition contenant des ions de calcium, selon lequel une couche de gel étant formée,
(vi) déposer l'apatite crystalline biomimétique sélectionnée de la fluoroapatite, l'hydroxyapatite ou leurs mélanges sur la surface du moulage prothétique, des composants organiques de l'agent gélifiant, de préférence de la gélatine, étant incorporés entre les couches de l'apatite ou dans des cavités, et
l'apatite ayant au moins une teneur en carbone dans la gamme de 0,25 à 2,5 % en poids et une teneur en azote dans la gamme de 0,09 à 0,9 % en poids.

10. Procédé selon la revendication 9, **caractérisé en ce que**
(a) la composition contenant des ions de phosphate comprend
(a.1) au moins un agent gélifiant,
(a.2) des phosphates hydrosolubles ou des phosphates étant hydrolysables à des ions de phosphate,
(a.3) facultativement le fluorure,
(a.4) facultativement un acide carboxylique ou un système tampon du pH 4 à 7,
(a.5) facultativement le glycérine, et
(b) la composition contenant des ions de calcium comprenant
(b.1) au moins un agent gélifiant,
(b.2) des ions de calcium,
(b.3) facultativement le glycérine.

11. Procédé selon la revendication 9 ou 10, **caractérisé en ce que** traiter la surface du moulage comprend le traitement mécanique, chimique, électrochimique et ou traiter dans un processus plasma ou par une combinaison des procédés, et une zone de la surface du moulage prothétique ayant des positions d'ancrage micromécaniques est obtenue, et facultativement la surface est activée par un processus chimique, électrochimique et/ou dans un processus plasma.

12. Procédé selon l'une des revendications 9 à 11, **caractérisé en ce que** la composition contenant des ions de phosphate et/ou des ions de calcium, chacune indépendamment, comprend des aminoacides, des dérivatifs des aminoacides, des protéines, du collagène ou du collagène dénaturé.

13. Procédé selon l'une des revendications 9 à 12, **caractérisé en ce que** l'agent gélifiant comprend la gélatine.

14. Procédé selon l'une des revendications 9 à 13, **caractérisé en ce que** la composition contenant des ions phosphate et/ou des ions de calcium, chacune indépendamment, a une teneur de l'eau.

15. L'intermédiaire, comprenant un moulage prothétique, au moins une zone de la surface du moulage ayant des positions d'ancrage micromécaniques, et le moulage ayant, au moins zone par zone, au moins une couche de gel d'une composition contenant des ions de phosphate sur sa surface, et facultativement là-dessus une autre couche de gel, et une couche de gel d'une composition contenant des ions de calcium,
- pour déposer l'apatite crystalline biomimétique sélectionnée de la fluoroapatite, l'hydroxyapatite ou leurs mélanges sur la surface du moulage prothétique, des composants organiques de l'agent gélifiant, de préférence de la gélatine, étant incorporés entre les couches de l'apatite ou dans des cavités, et l'apatite ayant au moins une teneur en carbone dans la gamme de 0,25 à 2,5 % en poids et une teneur en azote dans la gamme de 0,09 à 0,9 % en poids, pour produire un moulage prothétique selon l'une des revendications 1 à 8.

16. Utilisation de l'apatite crystalline biomimétique pour couvrir des moulages prothétiques, au moins zone par zone, et pour produire un moulage prothétique selon l'une des revendications 1 à 8.

17. Utilisation d'une composition contenant des ions de phosphate et d'une composition contenant des ions de calcium selon l'une des revendications 9 à 14, ou des formulations contenantes lesdites compositions, pour déposer biomimétiquement l'apatite sur une surface d'un moulage prothétique selon la revendication 1, la surface ayant des positions d'ancrage micromécaniques ou, la surface du moulage ayant été activée mécaniquement, chimiquement, électrochimiquement et/ou par moyen d'un processus plasma avant le dépôt, ou pour produire une intermédiaire selon la revendication 15.

18. Utilisation selon la revendication 17, **caractérisée en ce qu'**un dépôt de l'apatite biomimétique s'ensuit, au moins zone par zone, et/ou un dépôt essentiellement homogène de l'apatite s'ensuit dans ladite zone.

19. Moulage selon l'une des revendications 1 à 8 ou l'intermédiaire selon la revendication 15 pour l'utilisation pour le traitement dentaire, prothétique, ou chirurgical dans le cas de la perte de dent, en particulier pour l'attachement cellulaire des cellules des gencives ou des cellules muqueuses des gencives via des hémidesmosomes ou des autres mécanismes biologiques aux zones contactant le moulage.
